# FASCICULE DE BREVET EUROPEEN

(11) **EP 2 989 196 B1**
(45) Date de publication et mention de la délivrance du brevet: **09.05.2018**
(21) Numéro de dépôt: 14726772.8
(22) Date de dépôt: 24.04.2014
(51) Int. Cl.: C12N 1/12, C12R 1/89, B09C 1/10, C02F 3/32, C02F 3/34, C22B 3/18, C22B 3/24, C22B 60/02, G21F 9/18

(54) **NOUVELLE ALGUE RADIORESISTANTE DU GENRE COCCOMYXA**
NEUARTIGE STRAHLENRESISTENTE ALGEN DER GATTUNG COCCOMYXA
NOVEL RADIORESISTANT ALGA OF THE GENUS COCCOMYXA

(30) Priorité: 25.04.2013 FR 1353784
(43) Date de publication de la demande: 02.03.2016
(73) Titulaire: Commissariat à l'Énergie Atomique et aux Énergies Alternatives, 75015 Paris (FR); Institut Max Von Laue - Paul Langevin, 38042 Grenoble Cedex 9 (FR); Centre National de la Recherche Scientifique (C.N.R.S.), 75016 Paris (FR)
(72) Inventeur: RIVASSEAU, Corinne, F-38210 Cras (FR); FARHI, Emmanuel, F-38210 Cras (FR); ATTEIA, Ariane, F-13009 Marseille (FR); PRO, Danièle, F-38610 Gières (FR)
(74) Mandataire: Gevers & Orès
(86) Numéro de dépôt international: PCT/IB2014/060984
(87) Numéro de publication internationale: WO 2014/174483

(56) Documents cités:
- WO-A2-2011/098979
- CORINNE RIVASSEAU ET AL: "An extremely radioresistant green eukaryote for radionuclide bio-decontamination in the nuclear industry", ENERGY & ENVIRONMENTAL SCIENCE, vol. 6, no. 4, 1 avril 2013 (2013-04-01), pages 1230-1239, XP055088664, ISSN: 1754-5692, DOI: 10.1039/c2ee23129h -& DATABASE EMBL [Online] 1 novembre 2013 (2013-11-01), "Coccomyxa peltigerae genomic DNA containing 18S rRNA gene, ITS1, 5.8S rRNA gene, ITS2 and 26S rRNA gene, culture collection SAG:216-5", XP002716584, extrait de EBI accession no. EM_NEW:FN597599 Database accession no. FN597599 -& DATABASE EMBL [Online] 1 novembre 2013 (2013-11-01), "Coccomyxa chodatii genomic DNA containing 18S rRNA gene, ITS1, 5.8S rRNA gene, ITS2 and 26S rRNA gene, culture collection SAG:216-2", XP002729970, extrait de EBI accession no. EM_STD:FN597598 Database accession no. FN597598

## Description

L'invention est relative à de nouvelles algues et à leur utilisation pour le captage de métaux à partir de milieux aqueux, et notamment de milieux radioactifs.

Des effluents radioactifs sont produits principalement par les centrales de production d'électricité nucléaire. Il s'agit principalement des eaux des piscines d'entreposage de combustible usé, des eaux des bassins de décontamination, des eaux des circuits de refroidissement des installations nucléaires, ou encore des effluents ultimes rejetés dans l'environnement, qui finissent par contenir des composés radioactifs, du fait de l'activation de composés inactifs par les rayonnements, ou du relargage et de la dissolution de composés radioactifs. D'autres sources d'effluents radioactifs sont la médecine nucléaire, les hôpitaux réalisant des traitements radiochimiques, les laboratoires de recherche utilisant des matériaux radioactifs. Sont également concernés par la présente invention des effluents de certaines industries non nucléaires (par exemple l'extraction des terres rares).

Diverses méthodes physiques et chimiques sont utilisées pour purifier les effluents, notamment les eaux, contenant des composés radioactifs. Mais elles ont des coûts de fonctionnement et d'équipement importants, demandent une maintenance lourde et génèrent des volumes importants de déchets radioactifs. De plus, leur champ d'application est souvent limité. Par exemple, des résines échangeuses d'ions sont utilisées pour maintenir une faible conductivité dans les eaux des installations nucléaires. Elles se chargent en ions radioactifs et sont entreposées dans l'attente d'une filière de retraitement adaptée lorsqu'elles sont saturées ou stockées dans des conditions mettant en oeuvre des composés toxiques ou très réactifs.

Par ailleurs, il existe des méthodes biologiques utilisant par exemple des bactéries, des champignons, des levures ou des plantes pour purifier des milieux contaminés (effluents industriels, milieux naturels, etc.) par des produits toxiques (non radioactifs ou radioactifs). Ces méthodes utilisent des organismes vivants pour concentrer, assimiler, rendre moins toxiques (par modification de la forme chimique) des composés polluants ou de la biomasse non-vivante ainsi que des dérivés provenant d'organismes vivants pour biosorber les polluants.

Les méthodes biologiques ont en général un champ d'application plus large que les méthodes physiques et chimiques. Elles ne nécessitent pas l'ajout de réactif ou de produit chimique et permettent en général un traitement à plus faible coût, d'où leur intérêt économique.

Les plantes en particulier sont de bons épurateurs de sols ou d'eaux car elles possèdent tout un système de métabolites, protéines, enzymes, mécanismes d'import, canaux membranaires, structure interne, etc., qui les rendent capables selon les cas d'immobiliser des composés toxiques, de les chélater à l'extérieur ou à l'intérieur de la plante, de les incorporer en plus ou moins grande quantité via des voies d'import spécifiques ou non, de les séquestrer à l'intérieur des cellules, de modifier leur spéciation pour les rendre inoffensifs ou moins toxiques, de les rendre moins solubles, de les stocker sous forme non toxique dans la vacuole, etc.

Des études ont montré que certains micro-organismes étaient capables de concentrer par biosorption des ions métalliques, tels que Ag, Al, Au, Co, Cd, Cu, Cr, Fe, Hg, Mn, Ni, Pb, Pd, Pt, U, Th, Zn, etc., dans des solutions diluées (White et al., International Biodeterioration & Biodegradation, 35 : 17-40, 1995 ; Brevet US 6,355,172). La biosorption est la capacité de la biomasse à fixer des métaux lourds au moyen de mécanismes physico-chimiques, non actifs métaboliquement, par des interactions avec les groupements fonctionnels de composés pariétaux situés à la surface des cellules. Par exemple, il a été proposé d'utiliser des bactéries et des mélanges de micro-organismes pour biosorber non sélectivement des métaux lourds (Brevet US 7,479,220 ; Demande PCT WO 03/011487).

D'autres méthodes utilisent de la biomasse morte ou des composés dérivés provenant de la culture d'organismes vivants pour dépolluer des milieux contaminés en métaux. Les processus mis en jeu sont des mécanismes physico-chimiques, inactifs biologiquement, tels l'échange d'ions par exemple avec les polysaccharides présents dans les parois cellulaires, la complexation, ou l'adsorption.

La biomasse dérivée d'algues, par exemple de leurs parois cellulaires, a été utilisée pour épurer des métaux contenus dans des effluents liquides (Brevet US 4,769,223 ; Demande PCT WO 86/07346 ; Brevet US 5,648,313 ; Demande PCT WO 2006/081932).

Pour le traitement de milieux pollués par des composés radioactifs, les méthodes faisant appel à des organismes vivants sont peu nombreuses. En effet, dans le cas d'eaux contaminées par des composés radioactifs ou d'eaux situées au voisinage de sources radioactives, il est nécessaire d'utiliser des organismes radiotolérants ou radiorésistants, qui soient capables de résister à la toxicité chimique et radiologique des contaminants, mais aussi de fixer ces contaminants en quantité suffisante pour être utilisés dans le cadre d'un procédé industriel de décontamination.

Les procédés pour la biodécontamination des radionucléides dans les effluents nucléaires sont inexistants. Cependant, l'accident nucléaire de Fukushima du 11 mars 2011 a conduit le Japon à envisager ce type de solution à partir d'un matériau constitué d'une microalgue déshydratée, *Parachlorella* sp. *binos* (WO 2010/024367). Cinq grammes de cette microalgue ont permis de décontaminer 1 litre d'eau de composition similaire à celle des réacteurs de Fukushima. Les Etats-Unis d'Amérique s'intéressent depuis longtemps à la possibilité d'épurer les radionucléides par des méthodes biologiques. L'utilisation d'une algue, *Closterium moniliferum,* qui incorpore le strontium, a été envisagée, mais ni la capacité d'accumulation d'isotopes radioactifs ni la radiorésistance n'ont été testées pour cette algue (Krejci M.R. et al., ChemSusChem, 4:470-473, 2011; Lovett R.A., Nature, doi:10.1038/news.2011.195, 2011).

En milieu naturel, les organismes accumulant des composés radioactifs sont en général soumis à une radioactivité faible. Par exemple, immédiatement après l'accident de Tchernobyl, pour les milieux aquatiques, le débit de dose de rayonnements ionisants externe de l'eau du réservoir de refroidissement du réacteur n'a pas excédé 100 µGy/h et la dose maximale cumulée sur 1 an était de 0,01 Gy en 1986. Le débit de dose provenant des radionucléides déposés sur les sédiments dans la rivière Pripyat, située dans la zone de 30 km autour de la centrale, est quant à lui monté ponctuellement à 0,4 mGy/h immédiatement après l'accident (Kryshev et Sazykina, Journal of Environmental Radioactivity, 28 : 91-103, 1995).

Dans la plupart des cas, la résistance aux rayonnements ionisants des microorganismes proposés pour dépolluer des matériaux ou des effluents contaminés par la radioactivité et/ou concentrer des composés radioactifs n'a pas été testée. En effet, ils sont utilisés pour extraire l'uranium (U) et le thorium (Th) dont l'activité des principaux isotopes est faible. Par exemple, l'activité est respectivement de 0,13 et 0,8 Bq/l pour une solution contenant 10 µg/l de ²³⁸U ou ²³⁵U). Le Brevet US 4,320,093 propose l'utilisation de champignons du genre *Rhizopus* pour extraire de l'uranium ou du thorium contenu dans des effluents aqueux. Le Brevet GB 1,472,626 propose l'utilisation d'algues vertes unicellulaires mutantes obtenues par irradiation aux rayons X d'algues vertes unicellulaires préalablement accoutumées à l'uranium, et le Brevet GB 1,507,003 propose l'utilisation de divers microorganismes, notamment le champignon *Aspergillus niger* et des Cyanobactéries de type *Oscillatoria,* pour concentrer l'uranium naturellement présent dans l'eau de mer. Le Brevet US 7,172,691 propose l'utilisation d'algues vivantes photosynthétiques des genres *Chlorella, Scenedesmus, Oocystis,* et *Chlamydomonas,* pour concentrer des contaminants radioactifs, et notamment de l'uranium, à partir de milieux aqueux contenant une concentration d'uranium de l'ordre de 0-20 ppm, ce qui représente une activité de 260 et 1600 Bq/l pour ²³⁸U et ²³⁵U respectivement. En comparaison, l'activité de l'eau des piscines d'entreposage d'éléments nucléaires, qui constitue le milieu de vie de la microalgue selon l'invention est d'environ 300 000 Bq/l.

Les organismes les plus radiorésistants décrits jusqu'à présent sont des procaryotes. L'espèce *Deinococcus radiodurans* possède une extraordinaire capacité de résistance aux rayonnements ionisants qui se développe sous des irradiations de 60 Gy/h et survit à des doses de 15 kGy.

Mais il n'y a pas de lien entre radiotolérance et accumulation importante de radionucléides ou de métaux. L'utilisation la bactérie radiorésistante *Deinococcus radiodurans* comme épurateur de milieux radioactifs nécessite des modifications génétiques de la bactérie pour y introduire des gènes qui lui permettront d'accumuler les métaux d'intérêt. Ainsi, il a été proposé d'utiliser des bactéries du genre *Deinococcus* modifiées génétiquement pour exprimer des enzymes capables de détoxifier ou de métaboliser des composés organiques, des métaux, ou des radionucléides, à des fins de bioremédiation *in situ* de sites de déchets nucléaires (Demande PCT WO 01/23526). Plus récemment, des bactéries de l'espèce *Kineococcus radiotolerans* ont été isolées et purifiées à partir d'un site de déchets radioactifs de haute activité. Ces bactéries ont été décrites comme étant capables de dégrader des contaminants organiques en présence de rayonnements ionisants dont le débit de dose est supérieur à 10 Gy/h, et leur utilisation pour la dépollution non sélective de radionucléides par biosorption a été proposée mais non montrée (Brevet US 7,160,715).

Ces deux organismes présentent l'inconvénient d'être des bactéries non autotrophes, et donc de nécessiter l'apport extérieur de nutriments carbonés pour pouvoir utiliser leur culture. D'autre part, leur culture est plus sensible à la contamination que celle d'organismes autotrophes, ces derniers nécessitant un milieu de culture moins riche et moins sensible à la contamination bactérienne.

Deux organismes radiotolérants et autotrophes ont été décrits. Une microalgue de la classe des Chlorophycées, qui tolère des rayonnements ionisants avec une DL₅₀ de 6 kGy, a été étudiée (Farhi E. et al., J. Phys. Condens. Matt., 20 : 104216, 2008). Une nouvelle espèce de microalgue, *Coccomyxa actinabiotis,* a aussi été identifiée, et tolère des rayonnements ionisants avec une DL₅₀ de 10 kGy (Demande PCT WO 2011/098979). De tels niveaux de radiotolérance sont rares, notamment chez les algues. En général, les DL₅₀ de résistance aux rayonnements ionisants des algues sont comprises entre 30 et 1200 Gy (Agence internationale de l'énergie atomique, AIEA, « Effects of ionizing radioation on aquatic organisms and ecosystems », Technical reports series No. 172, 1976).

L'algue *Coccomyxa actinabiotis* décrite dans la Demande PCT WO 2011/098979 est capable de capter et de concentrer des ions métalliques radioactifs ou non en solution dans un milieu aqueux, et peut se développer en milieu radioactif.

Un test d'épuration d'une piscine grandeur réelle à base de cette algue a généré par exemple, pour une efficacité équivalente, un volume de déchets de l'ordre de 100 fois moins important que les résines échangeuses d'ions utilisées dans les procédés classiques d'épuration des eaux d'installation nucléaire (Rivasseau et al., Energy Environ. Sci., 6, 1230-1239 2013).

Les Inventeurs ont à présent découvert que d'autres microalgues du genre *Coccomyxa* sont capables de capter et de concentrer des ions radioactifs en solution dans un milieu aqueux, et qu'en outre elles peuvent se développer en milieu radioactif.

Ils ont en particulier découvert, dans les piscines de refroidissement d'éléments nucléaires usés, une nouvelle espèce de *Coccomyxa* se développant spontanément dans cet environnement riche en rayonnements ionisants et très pauvre en éléments nutritifs.

Ils ont isolé et caractérisé cette nouvelle espèce, dénommée ci-après *Coccomyxa* C-IR3-4C. Comme *Coccomyxa actinabiotis,* cette algue est à la fois radiotolérante et accumulatrice de radionucléides. Cette microalgue a été déposée selon le traité de Budapest le 29 janvier 2013 auprès de la *Culture Collection of Algae and Protozoa* (CCAP, *Scottish Association for Marine Science, Dunstaffnage Marine Laboratory,* GB-Oban, Argyll, PA37 1QA, Royaume-Uni) sous le numéro CCAP 216/26.

Les algues de l'espèce *Coccomyxa* C-IR3-4C se caractérisent notamment en ce que leurs gènes correspondant à l'ARN ribosomique 18S-ITS1-ARN ribosomique 5.8S-ITS2-ARN ribosomique 28S (début) contiennent une séquence présentant au moins 96%, et, par ordre de préférence croissante, au moins 97%, 98%, ou 99%, d'identité avec la séquence SEQ ID NO: 1.

Le pourcentage d'identité indiqué ci-dessus est calculé après alignement des séquences en utilisant l'outil BLAST®, sur une fenêtre de comparaison constituée par la totalité de la séquence SEQ ID NO: 1.

Les algues de l'espèce *Coccomyxa* C-IR3-4C peuvent également être caractérisées en ce que la région correspondant aux ITS1-ARNr5.8S-ITS2 présente au moins 96% d'identité avec la région correspondante de la séquence SEQ ID NO: 1. Ce seuil a été estimé à 96% sur la base des observations des Inventeurs, qui montrent un maximum de 95% d'identité entre cette région chez *Coccomyxa* C-IR3-4C et chez d'autres *Coccomyxae* ainsi qu'un maximum de 88% d'identité entre les ITS1-ARNr5.8S-ITS2 des autres *Coccomyxae* comparés entre eux.

La séquence des gènes d'ARN ribosomique de *Coccomyxa* C-IR3-4C se différencie notamment de celle d'autres espèces du genre *Coccomyxa* par la nature de ses gènes ITS1 et ITS2.

*Coccomyxa* C-IR3-4C peut croître en milieu radioactif, ce qui lui permet de capter et métaboliser des composés radioactifs autres que des métaux, en plus de pouvoir capter et concentrer des ions métalliques, radioactifs ou non, en solution dans un milieu aqueux radioactif ou non. Des composés radioactifs autres que des métaux sont par exemple ³H ou ¹⁴C. Ce dernier est métabolisable sous forme minérale de CO₂, carbonate ou hydrogénocarbonate au cours de la photosynthèse ou sous forme organique telle que l'acétate dans divers processus métaboliques des algues.

Les Inventeurs ont également découvert qu'une autre espèce de *Coccomyxa, Coccomyxa chodatii* (souche SAG n° 216-2 de la *Culture Collection of Algae at Goettingen University*), également radiotolérante, est capable de concentrer des radionucléides.

La présente invention a en conséquence pour objet l'utilisation d'algues vertes du genre *Coccomyxa,* et notamment de l'espèce *Coccomyxa* C-IR3-4C déposée sous le numéro CCAP 216/26, pour capter au moins un élément, radioactif ou non, choisi parmi l'antimoine (Sb) et les métaux suivants : Cs, Ag, Co, Mn, Sr, Cu, Cr, Zn, Ni, Fe, actinides, tels que l'uranium, lanthanides, et terres rares, telles que Sc, Y, La, Ce, Pr, Nd, Pm,
Sm, Eu, Gd, Tb, Dy, Ho, Er, Tm, Yb, Lu, et/ou pour capter au moins un des radioisotopes ¹⁴C et ³H, à partir d'un milieu aqueux contenant ledit élément et/ou ledit radioisotope en solution.

Plus particulièrement, la présente invention a pour objet un procédé de captage d'au moins un élément, radioactif ou non, choisi parmi Cs, Ag, Co, Mn, Sr, Cu, Cr, Zn, Ni, Fe, Sb, les actinides, les lanthanides et les terres rares, et/ou au moins un des radioisotopes ¹⁴C et ³H, à partir d'un milieu aqueux contenant ledit élément et/ou ledit radioisotope en solution, caractérisé en ce que ledit captage est effectué en incubant dans ledit milieu aqueux des algues vertes du genre C-IR3-4C déposée sous le numéro CCAP 216/26. De manière avantageuse, ledit élément est un métal choisi parmi Ag, Co, Cs, U, Mn, Cu et Sr, et les terres rares.

Selon un mode de réalisation particulier, l'invention concerne un procédé de captage d'au moins un élément, radioactif ou non, choisi parmi Sr et Cu à partir d'un milieu aqueux contenant ledit élément en solution, caractérisé en ce que ledit captage est effectué en incubant, dans ledit milieu aqueux, des algues vertes unicellulaires du genre *Coccomyxa.*

Selon un mode de mise en oeuvre préféré de la présente invention, ledit milieu aqueux est un milieu radioactif, c'est-à-dire un milieu dans lequel lesdites algues sont soumises à un débit de dose pouvant aller de quelques µGy/h jusqu'à quelques kGy/h. Selon une disposition préférée de ce mode de mise en oeuvre, l'élément à capter est un métal choisi parmi ceux indiqués ci-dessus, sous forme d'isotope stable ou radioactif, ou sous forme de mélange d'isotopes.

Selon un autre mode de mise en oeuvre préféré de la présente invention, ledit milieu aqueux est un milieu non radioactif. L'élément à capter est de préférence un métal choisi parmi les terres rares.

Selon un autre mode de réalisation de mise en oeuvre de la présente invention, ledit milieu aqueux est un milieu acide. Ceci est particulièrement avantageux, car les solutions de dissolution des déchets urbains sont généralement très acides (pH 1 ou pH 2). Les inventeurs ont montré (exemple 7.4) que les algues vertes unicellulaires *Coccomyxa* CCAP 216/26 étaient capables de survivre et de capter des terres rares à pH 1. L'élément à capter est de préférence un métal choisi parmi les terres rares. Les algues vertes unicellulaires du genre *Coccomyxa* peuvent donc être avantageusement utilisées, dans le cadre de la présente invention, pour la dépollution d'un milieu aqueux acide, de pH 1, de pH compris entre 1 et 2, de pH compris entre 2 et 3, ou de pH compris entre 3 et 6.

La durée d'incubation des algues dans le milieu aqueux peut varier notamment selon le ou les éléments concerné(s), d'une part, et la nature du milieu aqueux à partir duquel le captage doit être effectué. Elle sera généralement d'au moins 1 heure, et peut aller jusqu'à plusieurs mois, voire plusieurs années. Par exemple, si l'on souhaite capter Ag ou Cs ou U, une durée d'incubation d'environ 24 heures pourra être suffisante pour en capter la majeure partie.

La durée maximale d'incubation qui peut être envisagée dépendra en fait principalement de la capacité de croissance et de survie des algues dans le milieu aqueux.

En présence de lumière et de gaz carbonique (introduit par contact avec l'air ambiant, agitation des cultures ou bullage), les algues du genre *Coccomyxa,* et notamment de l'espèce *Coccomyxa* C-IR3-4C déposée sous le numéro CCAP 216/26, peuvent croître et vivre pendant de très longues périodes dans de l'eau faiblement minéralisée (conductivité 1 à 2 µS/cm), à un pH de 6 à 7 et une température de 20 à 30°C. Ces algues vertes ayant besoin de lumière pour réaliser la photosynthèse et fabriquer leur matière organique, leur croissance cesse lorsqu'elles sont placées à l'obscurité.

Ainsi, selon un mode de mise en oeuvre du procédé conforme à la présente invention, la croissance des algues vertes de l'espèce *Coccomyxa* C-IR3-4C déposée sous le numéro CCAP 216/26 peut être maîtrisée en contrôlant l'illumination du milieu aqueux comprenant lesdites algues.

Les algues *Coccomyxa* C-IR3-4C peuvent en outre croître et vivre pendant plusieurs années dans un milieu faiblement radioactif, où elles sont soumises à une irradiation inférieure ou égale à 0,1 mGy/h.

Pour la mise en oeuvre du procédé conforme à l'invention, les algues peuvent être utilisées en suspension dans le milieu aqueux à partir duquel le captage doit être effectué, sous agitation pour éviter leur agglomération. Elles peuvent également être fixées sur un support solide, lisse, poreux, ou sous forme de billes, placé dans ledit milieu aqueux.

Le procédé peut être déporté, c'est-à-dire que les algues sont mises en contact avec le milieu à dépolluer dans une enceinte séparée de l'installation nucléaire, ou mis en oeuvre *in situ;* dans ce dernier cas les algues sont alors implantées directement dans le milieu à dépolluer.

Dans le cadre de la présent invention les termes « dépollution » et « décontamination » sont synonymes et peuvent être employés l'un à la place de l'autre pour des éléments radioactifs ou non. Dans le cas d'un procédé de captage ou de décontamination *in situ,* les algues peuvent séjourner dans l'installation tant qu'elles ne gênent pas les opérations. A titre indicatif, leur croissance peut être maîtrisée par l'intensité d'éclairement (obscurité ou faible illumination), ou le choix de la longueur d'onde des lampes (par exemple lumière inactinique jaune-vert). Une filtration des eaux permet également de maîtriser leur croissance en captant les algues en suspension dans l'eau. S'il s'avère nécessaire de les éliminer, la destruction définitive des algues pourra être obtenue par oxydation, par exemple avec de l'eau oxygénée à 4 g/l pendant 1 à 7 jours. Cette opération s'accompagne d'un relargage des métaux, et devra donc préférentiellement s'effectuer par section dans les installations, avec récupération des effluents concentrés en métaux.

Dans le cas d'un procédé déporté ou d'un procédé *in situ,* à la fin de l'incubation, les algues ayant capté les métaux sont récoltées par des moyens conventionnels (filtration, décantation, centrifugation, etc.). Elles peuvent ensuite être éliminées comme des déchets éventuellement après avoir été séchées et/ou brûlées, sans extraction préalable des métaux qu'elles contiennent.

Avantageusement, on peut récupérer les éléments captés par les algues afin de recycler ces éléments. Cette récupération peut s'effectuer par tous moyens appropriés.

En particulier, les éléments peuvent être récupérés après destruction des algues. Cette destruction peut par exemple être effectuée par lyse des algues, avantageusement par oxydation, par exemple avec de l'eau oxygénée à 4 g/l pendant 1 à 7 jours. Elle peut aussi être effectuée par incinération des algues.

Les éléments peuvent être récupérés par ajout d'un complexant tel que l'EDTA (éthylène diamine tétraacétique acide) ou par ajout d'acide.

Le procédé conforme à l'invention peut être mis en oeuvre dans tous les cas où l'on souhaite extraire des éléments, radioactifs ou non, et notamment ceux mentionnés ci-dessus, d'un milieu aqueux, à des fins d'exploitation minière, ou de dépollution d'effluents aqueux, notamment d'effluents radioactifs.

Outre leur forte capacité à capter et concentrer spécifiquement les éléments mentionnés ci-dessus (Cs, Ag, Co, Mn, Sr, Cu, Cr, Zn, Ni, Fe, Sb, les actinides, les lanthanides et terres rares, telles que Sc, Y, La, Ce, Pr, Nd, Pm, Sm, Eu, Gd, Tb, Dy, Ho, Er, Tm, Yb, Lu, qu'ils soient radioactifs ou non), les algues du genre *Coccomyxa,* et notamment de l'espèce *Coccomyxa* C-IR3-4C déposée sous le numéro CCAP 216/26, possèdent également des propriétés de captage non-spécifique d'autres éléments, en particulier des métaux, notamment du fait de leur mucilage extracellulaire constitué de polysaccharides, qui ont la propriété de complexer les cations.

Le procédé conforme à l'invention est donc particulièrement approprié à la dépollution et à la décontamination de milieux aqueux ou de sols (tourbières, marais) contaminés par des métaux, radioactifs ou non, et plus particulièrement de milieux radioactifs, tels que l'eau des piscines de stockage ou encore l'eau légère des circuits de refroidissement secondaires de centrales ou de réacteurs nucléaires, ou les effluents de centrales nucléaires rejetés dans l'environnement, ou les effluents issus du milieu hospitalier.

La présente invention peut être mise en oeuvre non seulement avec des algues vertes unicellulaires de l'espèce *Coccomyxa,* C-IR3-4C, déposée sous le numéro CCAP 216/26, mais également avec un mélange de microorganismes comprenant au moins une algue verte unicellulaire appartenant au genre *Coccomyxa* C-IR3-4C déposée sous le numéro CCAP 216/26, et au moins un microorganisme, notamment une bactérie, un champignon, une levure, une autre algue unicellulaire, et/ou une plante multicellulaire, de préférence radiorésistant ou radiotolérant, capable de concentrer des ions métalliques en solution et/ou de capter et métaboliser des composés radioactifs autres que des métaux (par exemple ³H ou ¹⁴C). Les microorganismes et plantes multicellulaires utilisables en combinaison avec les algues vertes unicellulaires du genre *Coccomyxa* sont notamment ceux et celles cités ci-dessus, en particulier ceux et celles étant radiorésistants ou radiotolérants. Dans le cas où la présente invention est appliquée à un milieu aqueux radioactif, le temps d'incubation du mélange de microorganismes dépendra de la résistance individuelle des microorganismes composant le mélange. De même, les conditions de culture pourront être adaptées afin de favoriser la croissance d'un ou plusieurs microorganismes constituant le mélange.

La présente invention sera mieux comprise à l'aide du complément de description qui va suivre, qui se réfère à des exemples illustrant l'isolement et la caractérisation de l'espèce *Coccomyxa* C-IR3-4C, ainsi que son utilisation pour décontaminer un milieu aqueux radioactif, et la caractérisation des propriétés de captage de radionucléides de *Coccomyxa chodatii.*

### EXEMPLE 1 : ISOLEMENT ET CARACTERISATION DE COCCOMYXA C-IR3-4C

La microalgue a été récoltée dans une piscine de stockage d'éléments nucléaires usés. L'eau contenue dans cette piscine a un pH compris entre 6 et 7, une conductivité de 1 à 2 µS/cm, est en contact avec l'air ambiant et contient des éléments radioactifs dissous. Sa température varie entre 22 et 28°C et est en moyenne de 25°C. L'activité radiologique dans la piscine est très variable selon les points de mesure, faible à très forte.

La présence de films de matière organique verte a été observée sur les parois et diverses surfaces de cette piscine. Des prélèvements ont été effectués et leur observation au microscope a montré qu'il s'agissait d'une micro-algue verte unicellulaire.

### Conditions de culture

Les échantillons prélevés ont été stockés et mis en culture à la lumière, sur un milieu nutritif gélosé, dans un environnement stérile, à une température de 20 à 23°C. Le milieu nutritif est un milieu de culture BBM (Bold's Basal Medium, Sigma) pur ou dilué dans de l'eau déminéralisée. Le milieu BBM est classiquement utilisé pour la culture d'algues vertes. Le milieu de culture a un pH de 6,4. Sa composition est indiquée dans le Tableau I ci-dessous.

**Tableau I:**

| **Constituants en g/L** | **BBM** |
|---|---|
| NaNO3 | 0,25 |
| KH2PO4 | 0,175 |
| K2HPO4 | 0,075 |
| MgSO4, 7H2O | 0,075 |
| FeSO4, 7H2O | 0,005 |
| CaCl2, 2H2O | 0,025 |
| NaCl | 0,025 |
| Na2EDTA | 0,01 |
| KOH | 0,006 |
| H3BO4 | 12,86 |
| MnCl2, 4H2O | 1,81 |
| ZnSO4, 7H2O | 0,222 |
| Na2MoO4, 2H2O | 0,39 |
| CuSO4, 5H2O | 0,079 |
| Co(NO3)2, 6H2O | 0,049 |

Des algues ont été mises en culture sur un milieu de culture BBM solide gélosé. Des colonies ont été isolées, puis diluées de manière à étaler des cellules isolées sur milieu de culture gélosé. Cette opération a été répétée cinq fois afin d'obtenir la souche *Coccomyxa* C-IR3-4C qui a été séquencée.

Un échantillon de cette culture a été déposé selon le traité de Budapest le 29 janvier 2013 auprès de la *Culture Collection of Algae and Protozoa* (CCAP), sous le numéro CCAP 216/26.

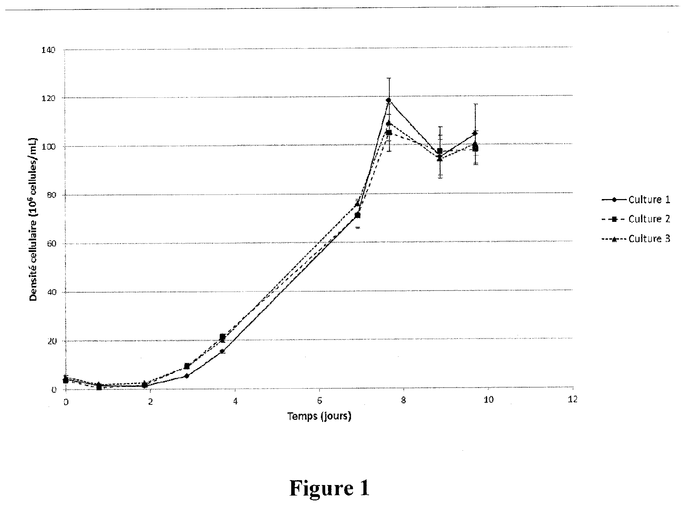

Dans un milieu BBM liquide, les microalgues de souche *Coccomyxa* C-IR3-4C ont cru avec une phase de croissance exponentielle. La croissance des microalgues a été mesurée par comptage de la densité cellulaire de la population algale au cours du temps sur trois échantillons d'algues cultivées en milieu BBM dilué deux fois, dans un incubateur Infors Multitron maintenu à 21±1°C, sous une agitation de 100 rpm et une illumination continue de 90±10 PAR. Le comptage est réalisé au moyen d'une cellule de Malassez sous microscope (grossissement objectif x40). Les courbes de croissance sont présentées sur la Figure 1, qui montre l'évolution de la densité cellulaire de la population algale en fonction de la durée de la culture.

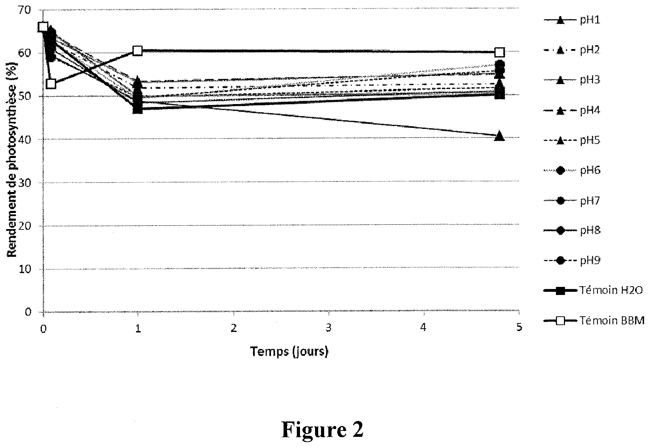

Les microalgues de souche *Coccomyxa* C-IR3-4C sont capables de vivre dans une large gamme de pH en milieu liquide. Les algues ont été cultivées dans de l'eau déminéralisée dont le pH a été ajusté aux valeurs cibles par ajout de HCl ou KOH. Les cultures sont réalisées dans un incubateur Infors Multitron maintenu à 21±1°C, sous une agitation de 100 rpm et une illumination continue de 90±10 PAR. Le pH des milieux est vérifié et réajusté quotidiennement si nécessaire. L'état des algues en fonction du pH du milieu est évalué par leur rendement de photosynthèse qui est un indicateur de l'état physiologique global des cellules. Le rendement de photosynthèse est mesuré en utilisant un fluorimètre PAM 103. La Figure 2 donne les valeurs du rendement de photosynthèse des microalgues en fonction de différents pH. Elle montre qu'elles peuvent vivre dans des milieux de pH 1 à pH 9 (gamme de pH testée). La Figure 2 indique que les microalgues conservent un bon état physiologique dans un milieu constitué d'eau déminéralisée et qu'elles conservent un bon état physiologique quel que soit le pH.

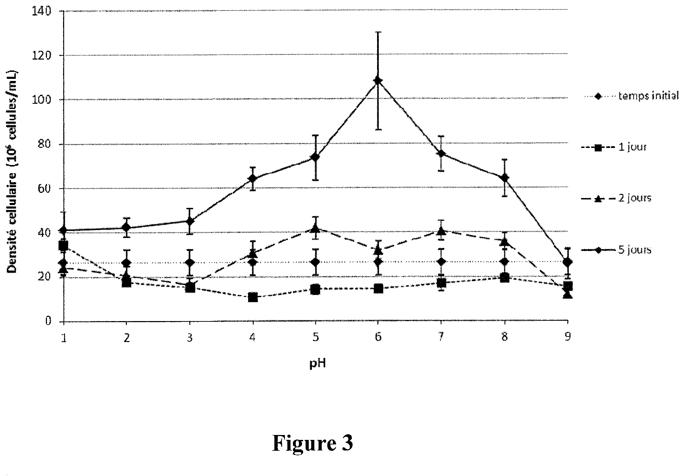

Les microalgues de souche *Coccomyxa* C-IR3-4C sont également capables de croître dans une large gamme de pH en milieu liquide. La Figure 3 montre l'évolution de la densité cellulaire de la population algale en fonction du pH au fil du temps dans des milieux de pH 1 à pH 9. Les conditions de culture sont identiques à celles de la Figure 2. Le comptage de la densité cellulaire est réalisé au moyen d'une cellule de Malassez sous microscope (grossissement x40). Pour obtenir une croissance rapide et une densité de microalgues importante, on pourra se placer dans la gamme de pH 4 à 8.

### Caractéristiques morphologiques et biochimiques

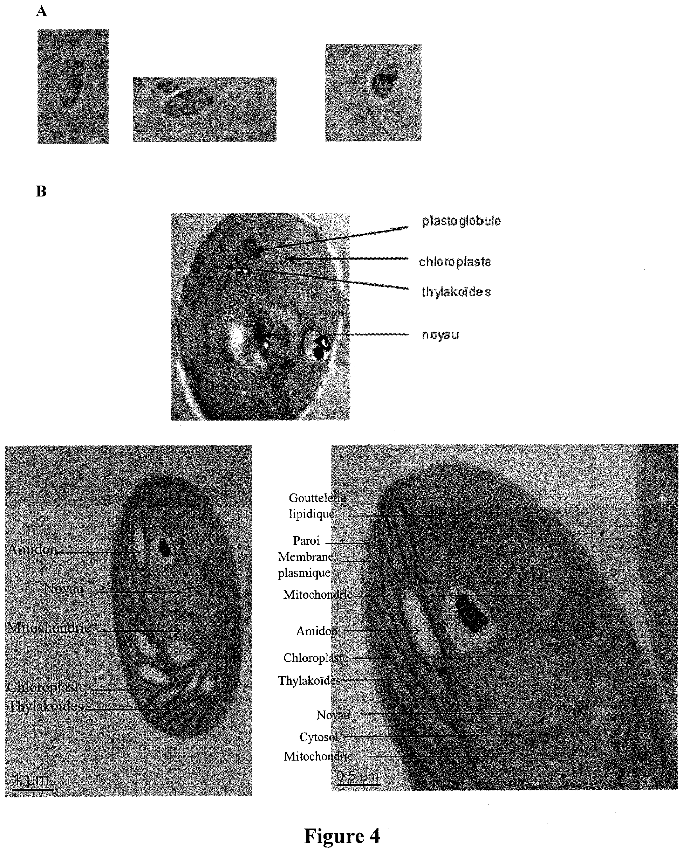

Les microalgues isolées observées en microscopie photonique et en microscopie électronique sont unicellulaires, ellipsoïdales et nucléées (Figures 4A et 4B). Les Figures 4A et 4B représentent des photographies de ces microalgues observées respectivement en microscopie photonique (microscope Zeiss binoculaire Axioplan 2, grossissement 1300) et en microscopie électronique à transmission (JEOL 1200EX et Philips CM 120. Les observations au TEM Philips CM 120 ont été réalisées au Centre Technologique des Microstructures - Plateforme de l'Université Claude Bernard Lyonl). Les microalgues renferment un chloroplaste (peut-être plusieurs) qui contient la chlorophylle et qui est le siège de la photosynthèse. D'autres organites, notamment la vacuole, occupent le reste de la cellule.

Le spectre d'absorption UV-visible de cet organisme montre la présence de chlorophylle a (pic d'absorption à 663nm), chlorophylle b (pic d'absorption à 647 nm) et de caroténoïdes (pic d'absorption à 470 nm).

### Amplification et séquençage des gènes de l'ADN ribosomal

L'ADN total de la microalgue C-IR3-4C isolée comme décrit ci-dessus a été extrait en utilisant le kit de purification d'ADN génomique « Wizard Genomic DNA purification Kit » (Promega).

La région du génome couvrant les gènes de l'ADN ribosomal ARNr18S-ITSl-ARNr5.8S-ITS2-ARNr28S (500 premières bases) a été amplifiée par PCR.

Les amorces utilisées sont EAF3 : TCGACAATCTGGTTG ATCCTGCCAG (SEQ ID NO: 2) et ITS055R : CTCCTTGGTC CGTGTTTCAAGACGGG(SEQ ID NO: 3), classiquement utilisées pour amplifier les gènes des ARNr de microalgues.

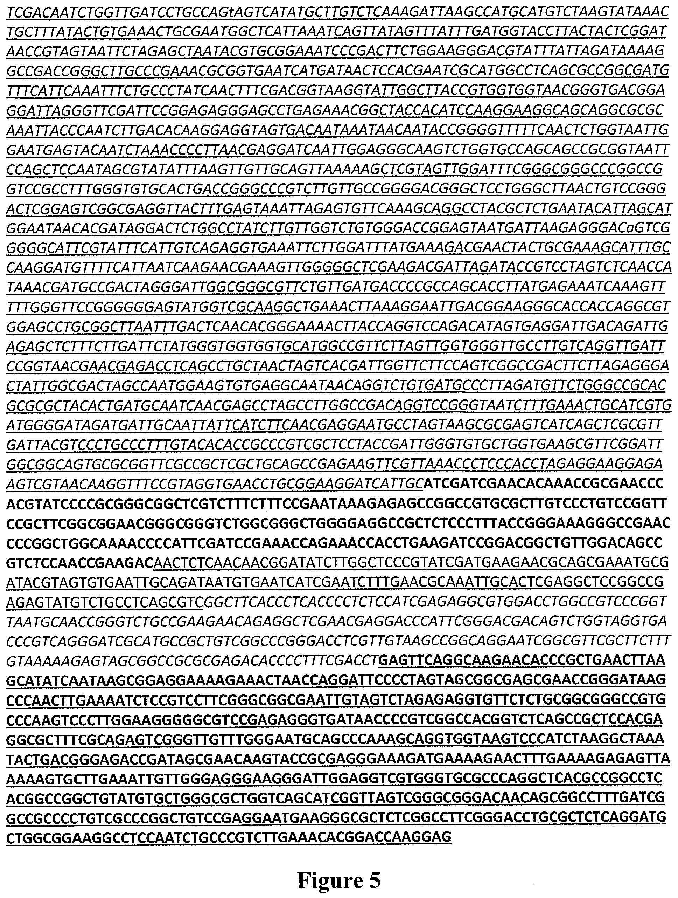

Les produits d'amplification obtenus en utilisant l'ADN isolé de deux cultures indépendantes ont été séquencés et sont de 3101 bases. La séquence de ces produits d'amplification est représentée à la Figure 5, ainsi que dans la liste des séquences en annexe sous le numéro SEQ ID NO: 1. Cette séquence représente la séquence de la région génomique couvrant les gènes de l'ADN ribosomal ARNr18S-ITS1-ARNr5.8S-ITS2-ARNr28S (début) de l'algue *Coccomyxa* C-IR3-4C. La partie correspondant à l'ARNr18S est en italique souligné, à ITS1 en gras, à l'ARNr5.8S en caractères soulignés, à ITS2 en italique, à l'ARNr28S en gras souligné.

L'algorithme BLASTN (Altschul et al., Nucleic Acids Research, 25 : 3389-3402, 1997) a été utilisé pour rechercher dans les bases de données les séquences de gènes d'ARN ribosomal présentant le maximum d'identité avec la séquence SEQ ID NO: 1. Cette recherche a fait apparaître que les espèces caractérisées les plus proches de la microalgue C-IR3-4C appartiennent au genre Coccomyxa.

La comparaison des séquences correspondant à l'ARN de la petite sous-unité du ribosome (ARNr 18S) de la microalgue C-IR3-4C (1807 bp), et des autres espèces de Coccomyxa répertoriées dans les bases de données a été réalisée par alignement multiple des séquences au moyen de l'algorithme BLAST®. Le Tableau II ci-dessous présente les résultats de cette comparaison de séquences. Les séquences des autres espèces sont accessibles dans la base de données GenBank, les numéros d'accès correspondants sont également indiqués dans le Tableau II.

**Tableau II :**

| Accession | Description | Total score | Query coverage | Max ident |
|---|---|---|---|---|
| HQ317304.1 | Coccomyxa rayssiae isolate UTEX273 small subunit ribosomal RNA gene, partial sequence | 3290 | 99% | 99% |
| FN298927.1 | Coccomyxa sp. CCAP 216/24 18S rRNA gene (partial), ITS1, 5.8S rRNA gene, ITS2 and 28S rRNA gene (partial), strain CCAP 216/24 | 3269 | 98% | 99% |
| FN298926.1 | Pseudococcomyxa simplex 18S rRNA gene (partial), ITS1, 5.8S rRNA gene, ITS2 and 28S rRNA gene (partial), strain SAG 216-9a | 3264 | 98% | 99% |
| HE586518.1 | Choricystis sp. GSE4G genomic DNA containing 18S rRNA gene, ITS1, 5.8S rRNA gene and ITS2, strain GSE4G | 3262 | 98% | 99% |
| FR865679.1 | 'Chlorella' saccharophila genomic DNA containing 18S rRNA gene, ITS1, culture collection CCAP 211/60 | 3262 | 98% | 99% |
| FN597598.1 | Coccomyxa chodatii SAG 216-2 | 3254 | 98% | 99% |
| FJ946891.1 | Trebouxiophyceae sp. VPL5-6 18S ribosomal RNA gene, partial sequence | 3234 | 97% | 99% |
| FJ648514.1 | Pseudococcomyxa simplex strain UTEX 274 18S ribosomal RNA gene, complete sequence | 3229 | 97% | 99% |
| FN597599.1 | Coccomyxa peltigerae SAG 216-5 | 3221 | 98% | 99% |
| HE586504.1 | Pseudococcomyxa simplex genomic DNA containing 18S rRNA gene, ITS1, 5.8S rRNA gene and ITS2, strain CAUP H 102 | 3195 | 96% | 99% |
| HE586513.1 | Coccomyxa sp. KN-2011-E4 genomic DNA containing 18S rRNA gene, ITS1, 5.8S rRNA gene and ITS2, strain E4 | 3181 | 96% | 99% |
| FN298928.1 | Coccomyxa sp. CCAP 211/97 18S rRNA gene (partial), ITS1, 5.8S rRNA gene, ITS2 and 28S rRNA gene (partial), strain CCAP 211/97 | 3181 | 98% | 99% |
| AB742451.1 | Coccomyxa sp. KGU-D001 gene for 18S ribosomal RNA, partial sequence | 3158 | 95% | 99% |
| HE586512.1 | Coccomyxa sp. KN-2011-C15 genomic DNA containing 18S rRNA gene, strain C15 | 3129 | 97% | 99% |
| FR850476.1 | Coccomyxa actinabiotis 18S rRNA, ITS1, 5.8S rRNA, ITS2 (CCAP 216-25) | 3126 | 98% | 99% |
| HE586505.1 | Pseudococcomyxa simplex genomic DNA containing 18S rRNA gene, strain CAUP H 103 | 3109 | 96% | 99% |
| HE586514.1 | Coccomyxa sp. KN-2011-T2 genomic DNA containing 18S rRNA gene, ITS1, 5.8S rRNA gene and ITS2, strain T2 | 3103 | 97% | 98% |
| AY422078.1 | Paradoxia multiseta 18S small subunit ribosomal RNA gene, partial sequence | 3081 | 93% | 99% |
| AM981206.1 | Coccomyxa sp. CPCC 508 18S rRNA gene, strain CPCC 508 | 3077 | 99% | 98% |
| AJ302939.1 | Coccomyxa sp. SAG 2325 18S rRNA gene, culture collection SAG:2325 | 3070 | 99% | 98% |
| AM167525.1 | Coccomyxa glaronensis 18S rRNA gene, strain CCALA 306 | 3061 | 99% | 97% |
| HE586507.1 | Ellipsoidion sp. UTEX B SNO113 genomic DNA containing 18S rRNA gene, strain UTEX B SNO113 | 3059 | 98% | 98% |
| HE586519.1 | Monodus sp. CR2-4 genomic DNA containing 18S rRNA gene, ITS1, 5.8S rRNA gene and ITS2, strain CR2-4 | 3055 | 98% | 98% |
| FR865588.1 | Chlamydomonas bipapillata genomic DNA containing 18S rRNA gene, ITS1, culture collection CCAP 11/47 | 3053 | 98% | 98% |
| EU282454.1 | Paradoxia sp. 294-GA206 18S ribosomal RNA gene, partial sequence | 3044 | 93% | 99% |
| GQ122371.1 | Trebouxiophyceae sp. KMMCC FC-10 18S ribosomal RNA gene, partial sequence | 3042 | 92% | 99% |
| HE586506.1 | Monodus sp. UTEX B SNO83 genomic DNA containing 18S rRNA gene, ITS1 and 5.8S rRNA gene, strain UTEX B SNO83 | 3035 | 98% | 97% |
| HE586509.1 | Coccomyxa sp. KN-2011-C10 genomic DNA containing 18S rRNA gene, strain C10 | 3022 | 97% | 98% |
| JQ946088.1 | Coccomyxa sp. XDL-2012 18S ribosomal RNA gene, partial sequence | 3014 | 92% | 99% |
| EU127471.1 | Coccomyxa sp. Flensburg fjord 2 18S ribosomal RNA gene, partial sequence | 3014 | 98% | 97% |
| FJ648513.1 | Coccomyxa mucigena strain SAG 216-4 18S ribosomal RNA gene, complete sequence | 2998 | 97% | 97% |
| HE586508.1 | Coccomyxa sp. KN-2011-C4 genomic DNA containing 18S rRNA gene, ITS1, 5.8S rRNA gene and ITS2, strain C4 | 2964 | 96% | 97% |
| EU127470.1 | Coccomyxa sp. Flensburg fjord 1 18S ribosomal RNA gene, partial sequence | 2953 | 97% | 97% |
| AY195970.1 | Choricystis sp. AS 5-1 18S ribosomal RNA gene, partial sequence | 2931 | 99% | 96% |
| AY197620.1 | Chlorella sp. Mary 9/21 BT-10w 18S ribosomal RNA gene, partial sequence | 2913 | 99% | 96% |
| AB080308.1 | Chlorella vulgaris gene for 18S rRNA, partial sequence | 2896 | 99% | 96% |
| EF106784.1 | Chlamydomonas sp. CCMP 681 | 2235 | 86% | 93% |

Cette comparaison de séquences montre que les espèces les plus proches de la souche C-IR3-4C sont *Coccomyxa* rayssiae souche UTEX273, *Coccomyxa* souche CCAP 216/24, *Pseudococcomyxa simplex* souche SAG 216-9a, *Coccomyxa chodatii* souche SAG 216-2, *Pseudococcomyxa simplex* souche UTEX 274, *Coccomyxa peltigerae* souche SAG 216-5, *Pseudococcomyxa simplex* souche CAUP H 102, *Coccomyxa* souche KN-2011-E4, *Coccomyxa* souche CCAP 211/97, ainsi que d'autres souches appartenant au genre des *Coccomyxae* avec 99% d'identité de séquence, puis d'autres souches appartenant toujours au genre des *Coccomyxae* telles que *Coccomyxa* souche CPCC 508 ou *Coccomyxa* souche SAG 2325 avec 98% d'identité de séquence, puis d'autres *Coccomyxae* telles que *Coccomyxa glaronensis* souche CCALA 306, *Coccomyxa* sp. Flensburg fjord 2 ou *Coccomyxa mucigena* souche SAG 216-4 avec 97% d'identité de séquence.

Ces forts scores d'identité obtenus pour la souche *Coccomyxa* C-IR3-4C par rapport au genre *Coccomyxa* sont voisins de ceux obtenus après comparaison des séquences des *Coccomyxae* entre elles (96-99%) et éloignés du score obtenu pour la comparaison de séquence avec une microalgue unicellulaire appartenant à un autre genre (*Chlamydomonas* sp. CCMP681 (EF106784.1), identité de 93%). Cela indique l'appartenance de la souche CCAP 216/26 au genre *Coccomyxa.*

Par ailleurs, la comparaison des séquences de la région des ITS de la souche C-IR3-4C avec celles des autres *Coccomyxae* a également été réalisée. Le Tableau III ci-dessous présente les résultats de cette comparaison de séquence des régions ITS1-ARNr5.8S-ITS2. Les séquences des autres espèces sont accessibles sur la base de données GenBank, les numéros d'accès correspondants sont également indiqués dans le Tableau III.

**Tableau III :**

| Accession | Description | Total score | Query coverage | Max ident |
|---|---|---|---|---|
| AY293967.1 | Coccomyxa solarinae var. saccatae internal transcribed spacer 1, 5.8S ribosomal RNA gene, and internal transcribed spacer 2, complete sequence | 1035 | 99% | 95% |
| AY293966.1 | Coccomyxa solarinae var. bisporae internal transcribed spacer 1, 5.8S ribosomal RNA gene, and internal transcribed spacer 2, complete sequence | 1030 | 99% | 95% |
| A Y293965.1 | Coccomyxa solarinae var. croceae internal transcribed spacer 1, 5.8S ribosomal RNA gene, and internal transcribed spacer 2, complete sequence | 1009 | 99% | 94% |
| HE586513.1 | Coccomyxa sp. KN-2011-E4 genomic DNA containing 18S rRNA gene, ITS1, 5.8S rRNA gene and ITS2, strain E4 | 1002 | 100% | 95% |
| HE586545.1 | Coccomyxa sp. KN-2011-E5 genomic DNA containing 18S rRNA gene, ITS1, 5.8S rRNA gene and ITS2, strain E5 | 985 | 99% | 94% |
| HE586551.1 | Coccomyxa sp. KN-2011-T5 genomic DNA containing ITS1, 5.8S rRNA gene and ITS2, strain T5 | 939 | 93% | 94% |
| AY293964.1 | Coccomyxa peltigerae var. variolosae internai transcribed spacer 1, 5.8S ribosomal RNA gene, and internal transcribed spacer 2, complete sequence | 910 | 94% | 94% |
| HQ335215.1 | Coccomyxa sp. S2 internal transcribed spacer 1, partial sequence; 5.8S ribosomal RNA gene, complete sequence; and internai transcribed spacer 2, partial sequence | 745 | 83% | 92% |
| HQ335216.1 | Coccomyxa sp. S10 internal transcribed spacer 1, partial sequence; 5.8S ribosomal RNA gene, complete sequence; and internai transcribed spacer 2, partial sequence | 745 | 83% | 92% |
| FN298926.1 | Pseudococcomyxa simplex 18S rRNA gene (partial), ITS1, 5.8S rRNA gene, ITS2 and 28S rRNA gene (partial), strain SAG 216-9a | 673 | 92% | 95% |
| HE586504.1 | Pseudococcomyxa simplex genomic DNA containing 18S rRNA gene, ITS1, 5.8S rRNA gene and ITS2, strain CAUP H 102 | 668 | 92% | 94% |
| FN298927.1 | Coccomyxa sp. CCAP 216/24 18S rRNA gene (partial), ITS1, 5.8S rRNA gene, ITS2 and 28S rRNA gene (partial), strain CCAP 216/24 | 651 | 100% | 88% |
| AY328524.1 | Coccomyxa rayssiae strain SAG 216-8 18S ribosomal RNA gene, partial sequence; internai transcribed spacer 1, 5.8S ribosomal RNA gene and internal transcribed spacer 2, complete sequence; and 26S ribosomal RNA gene, partial sequence | 640 | 92% | 94% |
| HE586526.1 | Pseudococcomyxa sp. KN-2011-A1 genomic DNA containing ITS1, 5.8S rRNA gene and ITS2, intragenomic variability copy B, strain A1 | 568 | 90% | 91% |
| HE586525.1 | Pseudococcomyxa sp. KN-2011-A1 genomic DNA containing ITS1, 5.8S rRNA gene and ITS2, intragenomic variability copy A, strain A1 | 566 | 90% | 91% |
| AY293968. 1 | Coccomyxa chodatii internai transcribed spacer 1, 5.8S ribosomal RNA gene, and internai transcribed spacer 2, complete sequence | 559 | 92% | 90% |
| FR850476.1 | Coccomyxa actinabiotis rRNA ITS1-5.8S-ITS2-28S | 376 | 59% | 88% |
| AY328522.1 | Coccomyxa peltigerae strain SAG 216-5 18S ribosomal RNA gene, partial sequence; internai transcribed spacer 1, 5.8S ribosomal RNA gene and internal transcribed spacer 2, complete sequence; and 26S ribosomal RNA gene, partial sequence | 366 | 31% | 94% |
| HE586515.1 | Coccomyxa sp. KN-2011-T3 genomic DNA containing 18S rRNA gene, ITS1, 5.8S rRNA gene and ITS2, strain T3 | 158 | 23% | 83% |
| HE586550.1 | Coccomyxa sp. KN-2011-T1 genomic DNA containing 18S rRNA gene, ITS1, 5.8S rRNA gene and ITS2, strain T1 | 158 | 23% | 83% |
| HE586514.1 | Coccomyxa sp. KN-2011-T2 genomic DNA containing 18S rRNA gene, ITS1, 5.8S rRNA gene and ITS2, strain T2 | 128 | 15% | 86% |

Cette comparaison de séquences montre une identité de 83% à 95% de la séquence de la souche C-IR3-4C par rapport aux autres souches *Coccomyxae* référencées, du même ordre que les scores obtenus pour la comparaison de la région des ITS des *Coccomyxae* référencées entre eux (78% à 99%), et très éloigné de celui obtenu pour la comparaison avec d'autres genres (de l'ordre de 30 à 50%).

Il s'agit donc d'une espèce de *Coccomyxa* différente de toutes celles référencées à ce jour. Ces résultats indiquent en effet que la microalgue isolée appartient au genre *Coccomyxa,* mais que son ADN diffère suffisamment de celui des autres espèces de *Coccomyxae* répertoriées dans les banques de données, notamment au niveau de l'ADN des ITS1 et ITS2 pour que l'on considère qu'il s'agit d'une nouvelle espèce, qui est dénommée ici *Coccomyxa* C-IR3-4C.

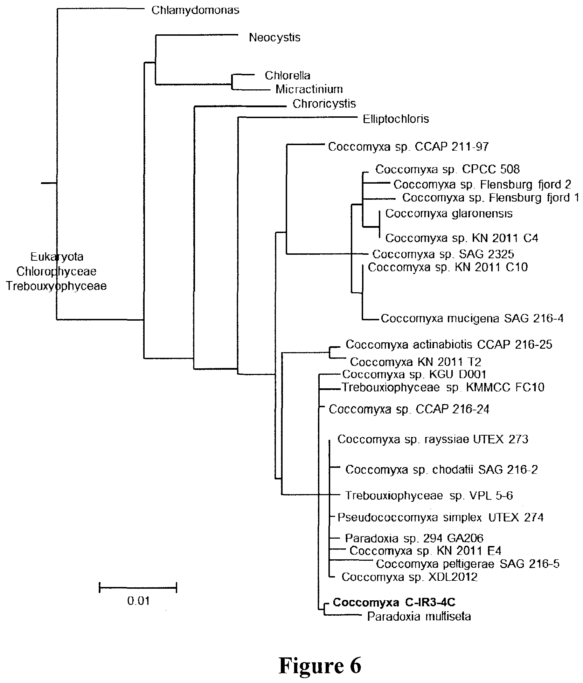

La Figure 6 présente l'arbre phylogénétique obtenu après alignement des séquences d'ADN correspondant à l'ARNr 18S (BLASTN). La barre indique un taux de substitution de 1% (0,01).

### EXEMPLE 2: RESISTANCE AUX RAYONNEMENTS IONISANTS DE COCCOMYXA C-IR3-4C

La résistance aux rayonnements ionisants des algues *Coccomyxa* C-IR3-4C a été testée en les exposant à différentes doses de rayonnements gamma issus d'éléments de combustible usés en cours de décroissance. La mortalité après irradiation a été déterminée par colorant vital (rouge neutre).

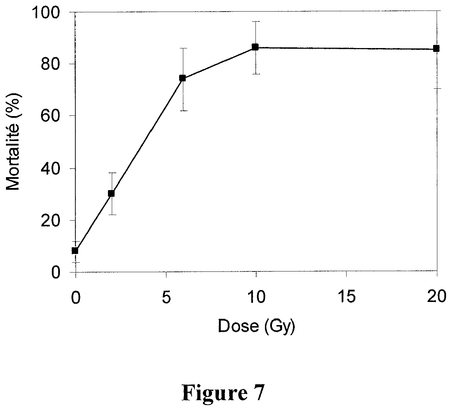

La Figure 7 montre le pourcentage de mortalité de *Coccomyxa* C-IR3-4C en fonction de la dose d'irradiation, mesuré trois jours après l'irradiation aiguë. La dose de rayonnements ionisants qui provoque la mort de la moitié de la population est comprise entre 2 et 6 kGy.

### EXEMPLE 3 : DECONTAMINATION D'EAUX D'INSTALLATIONS NUCLEAIRES AU MOYEN DE COCCOMYXA C-IR3-4C

Une eau de composition typique de celle d'eaux provenant d'une piscine de stockage d'éléments nucléaires usés a été mise en contact avec *Coccomyxae* C-IR3-4C pendant 24 h, sous illumination. Une masse fraîche de 100 mg d'algues *Coccomyxae* C-IR3-4C initialement cultivées en milieu liquide BBM et préalablement rincées trois fois avec de l'eau milli-Q est mise en contact avec 50 ml d'une eau contenant initialement les radionucléides émetteurs bêta ³H (200000 Bq/l) et ¹⁴C (1000 Bq/l), ainsi que les radionucléides émetteurs gamma listés dans le Tableau IV ci-dessous.

**Tableau IV:**

| **Radionucléide** | ⁵⁴Mn | ⁶⁰Co | ^{110m}Ag | ¹³⁷Cs | ²³⁸U |
|---|---|---|---|---|---|
| **Activité (Bq/l)** | 9 | 42 | 23 | 67 | 21 |

Le dosage de l'eau et des algues par spectrométrie γ 24 h après la mise en contact montre une élimination de 81% de l'activité y de l'eau.

Le pourcentage de radionucléides émetteurs γ fixés par les algues en 24 h est indiqué dans le tableau V ci-dessous.Tableau V:

| **Radionucléide** | ⁵⁴Mn | ⁶⁰Co | ^{110m}Ag | ¹³⁷Cs | ²³⁸U |
|---|---|---|---|---|---|
| **% de fixation** | 52 | 45 | 100 | 95 | 100 |

La totalité ou quasi-totalité de l'^{110m}Ag, du ¹³⁷Cs et de l'²³⁸U et la moitié du ⁵⁴Mn et du ⁶⁰Co ont été épurés de l'eau.

### EXEMPLE 4 : DECONTAMINATION D'UNE EAU DE PISCINE D'ENTREPOSAGE AU MOYEN DE COCCOMYXA C-IR3-4C PAR ACTION IN SITU

Les microalgues *Coccomyxae* C-IR3-4C, présentes dans une piscine de refroidissement d'éléments nucléaires usés, décontaminent les radionucléides présents dans cette piscine. Cette piscine est remplie d'eau de pH compris entre 6 et 7, de conductivité comprise entre 1 et 2 µS/cm, de température moyenne 25°C, et contient des éléments métalliques radioactifs du fait des pièces qui y sont entreposées. Elle est en contact avec l'air ambiant et est illuminée par des néons.

Dans ces conditions, *Coccomyxa* C-IR3-4C est capable de coloniser les supports de la piscine et peut y vivre et s'y reproduire pendant des années.

Des comptages réalisés par spectrométrie y ont permis de déterminer l'activité totale ainsi que la nature et l'activité de chaque émetteur y concentrés par environ 1 g de masse fraîche d'algues et sont donnés dans le Tableau VI ci-dessous. L'activité des microalgues est définie dans ce tableau comme le rapport de l'activité (Bq) d'1g de masse fraiche d'algues à l'activité d'1 mL de l'eau dans laquelle elles vivent.

**Tableau VI:**

| **Radionucléide** | **Activité des microalgues/Activité de l'eau** |
|---|---|
| ⁶⁰Co | 67 |
| ⁶⁶Cu | 167 |
| ⁹²Sr | 7,5 |
| ¹¹⁰mAg | 62 |
| ¹³⁴Cs | 17 |
| ¹³⁷Cs | 8030 |
| Total | 8380 |

Ces résultats montrent que les algues sont jusqu'à 10⁴ fois plus actives que l'eau dans laquelle elles vivent, et donc qu'elles ont effectivement concentré les radioéléments ⁶⁰Co, ⁶⁶Cu, ⁹²Sr, ^{110m}Ag, ¹³⁴Cs et ¹³⁷Cs.

Le facteur de concentration du césium 137 (¹³⁷Cs) par l'algue *Coccomyxa* C-IR3-4C, défini comme le rapport de la concentration de Cs fixé par les algues, en atomes/g de matière fraîche, à la concentration de Cs dans l'eau, en atomes/ml, est de l'ordre de 20000.

### EXEMPLE 5 : DECONTAMINATION D'EAUX D'INSTALLATIONS NUCLEAIRES AU MOYEN DE COCCOMYXA CHODATII

La détermination de la résistance aux rayonnements ionisants des algues *Coccomyxa chodatii* (souche SAG 216/2) montre que la dose de rayonnements ionisants qui provoque la mort de la moitié de la population est de l'ordre de 1.5 kGy (Rivasseau *et al.,* 2013, précité). La DL₅₀ de résistance aux rayonnements ionisants des algues est généralement comprise entre 30 et 1200 Gy (AIEA, 1976, précité).

Il semble que les microalgues appartenant au genre *Coccomyxa* présentent une radiotolérance supérieure à celles d'algues appartenant à d'autres genres, avec une résistance exceptionnelle chez *Coccomyxa actinabiotis,* de la même manière que toutes les bactéries appartenant au genre *Deinococcus* présentent une forte radiotolérance.

Afin de déterminer si *Coccomyxa chodatii* partageait la capacité de captage d'éléments de *Coccomyxa actinabiotis* et *Coccomyxa* C-IR3-4C, une eau de composition typique de celle d'eaux provenant d'une piscine de stockage d'éléments nucléaires usés a été mise en contact avec des *Coccomyxae chodatii* pendant 24 h, sous illumination. Une masse fraîche de 110 mg d'algues *Coccomyxae chodatii* initialement cultivées en milieu liquide BBM et préalablement rincées trois fois avec de l'eau milli-Q est mise en contact avec 50 ml d'une eau contenant initialement les radionucléides émetteurs bêta ³H (200000 Bq/l) et ¹⁴C (1000 Bq/l), ainsi que les radionucléides émetteurs gamma listés dans le Tableau IV ci-dessus.

Le dosage de l'eau et des algues 24 h après la mise en contact montre une élimination de 80% de l'activité γ de l'eau.

Le pourcentage de radionucléides émetteurs y fixés par les algues en 24 h est indiqué dans le tableau VII ci-dessous.

**Tableau VII:**

| **Radionucléide** | ⁵⁴Mn | ⁶⁰Co | ^{110m}Ag | ¹³⁷Cs | ²³⁸U |
|---|---|---|---|---|---|
| **% de fixation** | 100 | 54 | 100 | 94 | 100 |

La totalité ou quasi-totalité de l'^{110m}Ag, du ⁵⁴Mn, du ¹³⁷Cs et de l'²³⁸U et la moitié du ⁶⁰Co ont été épurés de l'eau.

### EXEMPLE 6: MAITRISE DE LA PROLIFERATION OU ELIMINATION DES MICROALGUES

Les microalgues sont photosynthétiques. Elles ont besoin de lumière pour réaliser la photosynthèse et fabriquer leur matière organique. Dans le milieu très pauvre en nutriments qui correspond aux divers milieux et effluents des centrales nucléaires, leur croissance peut donc être maîtrisée par l'illumination. Pour qu'elles se développent à un endroit donné, il suffit de les éclairer. Leur croissance peut aussi être maîtrisée en les éclairant avec une lumière qui ne permette pas ou peu de photosynthèse, par exemple avec une lampe inactinique de couleur jaune-verte.

Une filtration des eaux permet de capter les algues en suspension dans l'eau, et d'en maîtriser leur croissance.

Une méthode chimique telle une oxydation, par exemple par l'eau oxygénée, permettra de les éliminer totalement. Dans un milieu contenant 20 ml de suspension cellulaire de microalgues, on introduit 5 ml d'eau oxygénée à 20 g/l, soit une concentration finale de H₂O₂ de 4 g/l. Après 1 jour, la culture contient des agrégats de matière marron-blanc et sa couleur verte a disparu. Au bout d'une semaine, plus rien n'est observable au microscope.

La dégradation des algues par l'eau oxygénée est donc rapide, progressive et totale, ne laissant aucun résidu de matière organique.

Cette solution peut aussi être employée pour nettoyer des pièces radioactives transférées d'un milieu à un autre et éviter toute contamination du nouveau milieu par des algues, ou encore pour nettoyer les parois des piscines lorsque celles-ci sont vidées.

### EXEMPLE 7: BIO-PROCEDES DE CAPTAGE DE TERRES RARES PAR COCCOMYXA CCAP 216/26

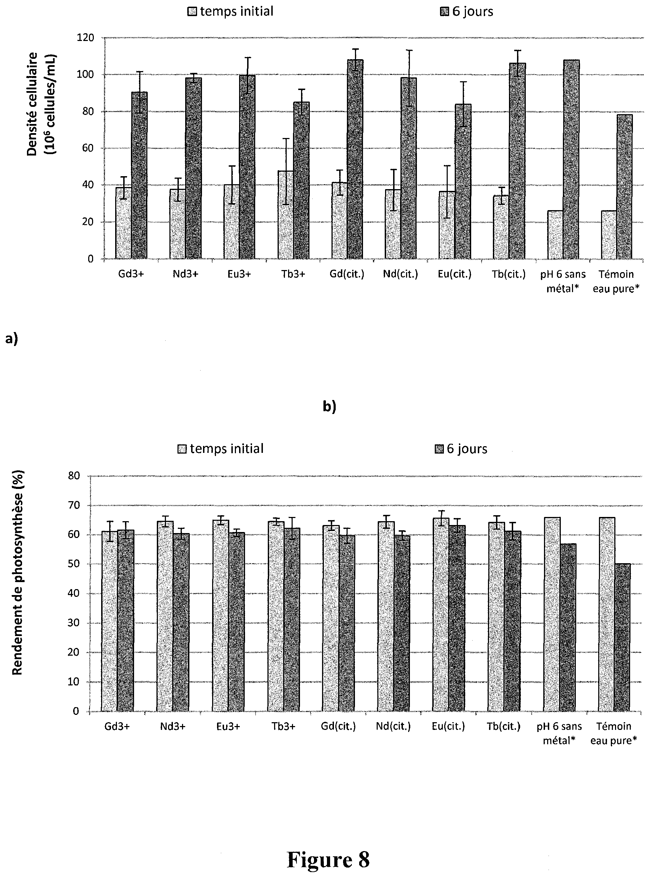

Cet exemple fait référence aux Figures 8 à 13, dont les légendes sont présentées ci-dessous :
**Figure 8** **:** Evolution **a)** de la densité cellulaire et **b)** du rendement de photosynthèse de micro-algues *Coccomyxa* CCAP 216/26 exposées à 10⁻⁶ M de terres rares à pH 6, conditions optimales pour la bio-épuration (n=3 réplicats biologiques). Quatre terres rares (Gd, Nd, Eu et Tb) et deux formes chimiques (cation et complexes du citrate) ont été testées en parallèle. * = échantillons témoins non réalisés en parallèle et non répliqués. Ces valeurs sont introduites à titre indicatif pour leur pertinence. Le second prélèvement n'a pas eu lieu après 6 jours d'exposition, mais après 5 jours. Le pH dans l'eau pure est de 7 ± 1.

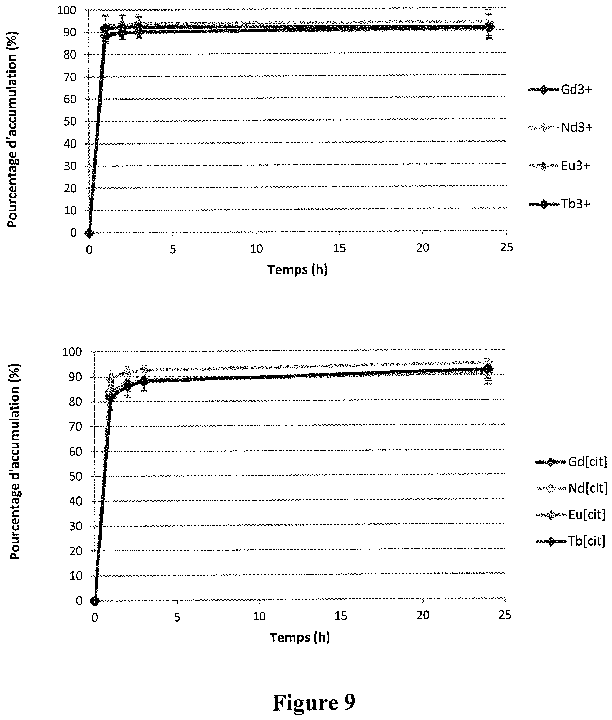

**Figure 9** **:** Pourcentages de terres rares accumulées (%) par des micro-algues *Coccomyxa* CCAP 216/26 exposées à 10⁻⁶ M de métal à pH 6, conditions optimales pour la bio-épuration (n=3 réplicats biologiques). Quatre terres rares (Gd, Nd, Eu et Tb) et deux formes chimiques (cation et complexes du citrate) ont été testées en parallèle (moyenne ± écart type).

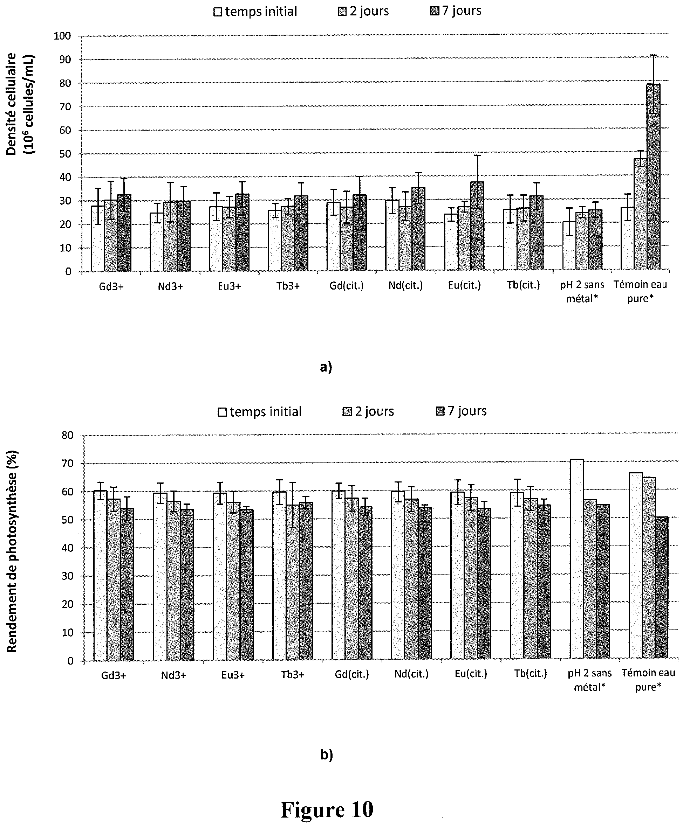

**Figure 10** **:** Evolution **a)** de la densité cellulaire et **b)** du rendement de photosynthèse de micro-algues *Coccomyxa* CCAP 216/26 exposées à 10⁻² M de terres rares à pH 2, conditions optimales pour le captage de métaux (n=5 réplicats biologiques). Quatre terres rares (Gd, Nd, Eu et Tb) et deux formes chimiques (cation et complexes du citrate) ont été testées en parallèle. * = échantillons témoins non réalisés en parallèle et non répliqués. Ces valeurs sont introduites à titre indicatif pour leur pertinence. Le troisième prélèvement n'a pas eu lieu après 7 jours d'exposition, mais après 5 jours. Le pH dans l'eau pure est de 7 ± 1.

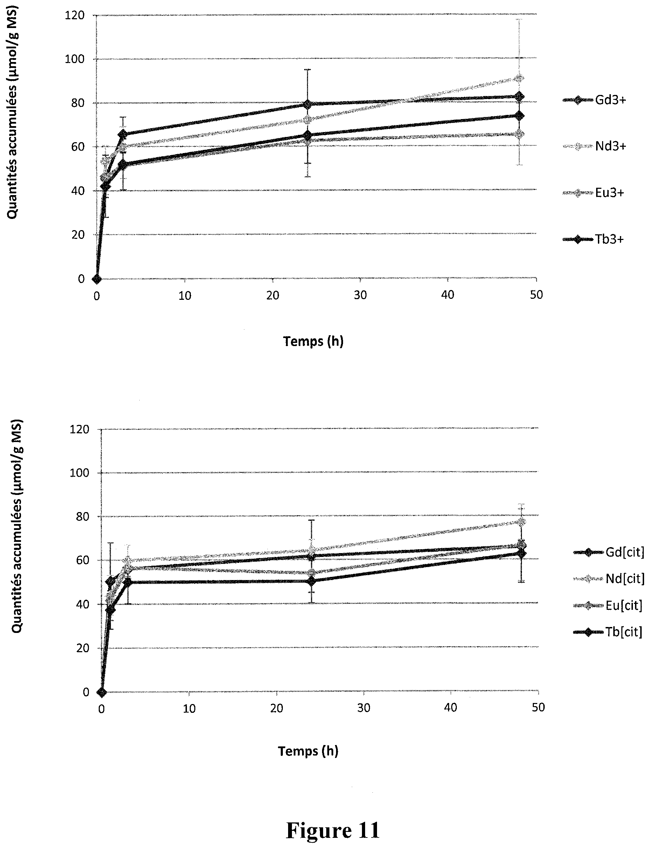

**Figure 11** : Quantités de terres rares accumulées (µmol/g de matière sèche) par des micro-algues *Coccomyxa* CCAP 216/26 exposées à 10⁻² M de métal à pH 2, conditions optimales pour le captage de métaux (n=3 réplicats biologiques). Quatre terres rares (Gd, Nd, Eu et Tb) et deux formes chimiques (cation et complexes du citrate) ont été testées en parallèle.

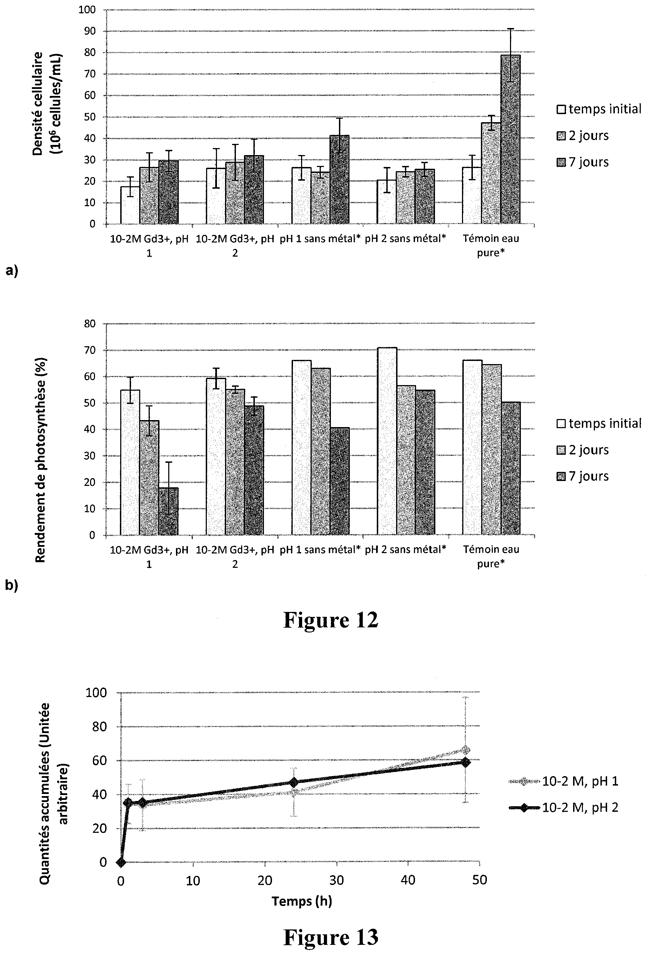

**Figure 12** **:** Evolution a) de la densité cellulaire et b) du rendement de photosynthèse de micro-algues *Coccomyxa* CCAP 216/26 exposées à 10⁻² M de métal et à pH 1 ou 2 (n=5 réplicats biologiques). Les deux conditions d'exposition ont été testées en parallèle. * = échantillons témoins non réalisés en parallèle et non répliqués. Ces valeurs sont introduites à titre indicatif pour leur pertinence. Le troisième prélèvement n'a pas eu lieu après 7 jours d'exposition, mais après 5 jours. Le pH dans l'eau pure est de 7 ± 1.
**Figure 13** **:** Quantités de terres rares accumulées (Unité arbitraire) par des micro-algues *Coccomyxa* CCAP 216/26 exposées à 10⁻² M de métal et à pH 1 ou 2 (n=3 réplicats biologiques dans les conditions 10⁻² M de Gd3+ et pH 1 ; n=2 réplicats biologiques dans les conditions 10⁻² M de Gd3+ et pH 2). Les deux conditions d'exposition ont été testées en parallèle.

### 7.1. Protocoles expérimentaux

### 7.1.1. Matériel et méthodes

Pour respecter les conditions stériles lors de la culture des algues, les manipulations ont été réalisées sous hotte à flux laminaire, dans la zone de stérilité d'une flamme. Le matériel et les solutions utilisés ont été préalablement stérilisés par autoclave. Les récipients de culture ont été recouverts avec du papier aluminium, afin de permettre les échanges gazeux. En revanche, les expériences d'exposition des algues aux métaux ont été réalisées en conditions non stériles.

Les centrifugations ont été réalisées à l'aide une centrifugeuse de type Megafuge 16R (Thermo Scientific) équipée d'un rotor TX-400 (Thermo Scientific). L'utilisation d'adaptateurs adéquats (réf. 75003683 et 75003682, Thermo Scientific) a permis de centrifuger des échantillons contenus dans des tubes Falcon stériles à usage unique de 15 ou 50 mL (réf. 734-0451 et 734-0448, VWR).

Les micro-algues ont été observées à l'aide d'un microscope optique de type Optiphot (Nikon) équipé d'objectifs de grossissement x20 et x40. La densité cellulaire a été mesurée par comptage de 12 µL de suspension d'algues sur des cellules de Malassez aluminées de 0,0025 mm², profondeur 0,200 mm (réf 0640630, Marienfeld). Une dilution préalable des échantillons a été réalisée si besoin, de manière à compter entre 50 et 100 cellules par case.

Le rendement de photosynthèse a été mesuré à l'obscurité à l'aide d'un fluorimètre à chlorophylle de type PAM-103 (Walz).

Les terres rares ont été dosées dans les liquides et dans les algues par spectrométrie de masse couplée à une torche à plasma, sur un appareillage de type Hewlett-Packard 4500 ICP-MS System. Les protocoles utilisés et les isotopes sélectionnés sont décrits au paragraphe 7.1.4.

### 7.1.2. Mise en oeuvre des algues

### Repiquage en milieu liquide

Les algues en suspension ont été récoltées par centrifugation dans des conditions douces (100 g, 25 min, 4°C, accélération et décélération 2). Les algues contenues dans le culot ont ensuite été lavées par ajout d'eau milli-Q stérile suivi d'une centrifugation, avant d'être remises en suspension dans un nouveau milieu de culture stérile, à une densité cellulaire initiale de 5.10⁶ cellules/mL.

### Culture des algues

Les algues *Coccomyxa* CCAP 216/26 ont été cultivées en photo-bioréacteur de 2 L, dans 1,5 L de milieu de culture stérile consistant en un milieu BBM (B5282, Sigma-Aldrich) dilué par deux dans de l'eau milli-Q (0,5x). La température et la luminosité ont été maintenues constantes, respectivement à 22 ± 1 °C et 90 ± 10 µmol/m²/s. L'agitation et l'aération des cellules ont été assurées par un bullage d'air continu. La concentration initiale des algues est de l'ordre de 5.10⁶ cellules/mL. Un ajout de 15 mL de milieu BBM concentré (50x) a été réalisé tous les 3 à 4 jours à partir de la deuxième semaine de culture pour nourrir les cellules. Le photo-bioréacteur a été utilisé en mode batch et a été renouvelé chaque mois. Les algues ont été utilisées dès lors que la densité cellulaire maximale, supérieure à 100.10⁶ cellules/mL, a été atteinte (phase plateau alimenté).

### Récolte et lavage des algues

Après une récolte par centrifugation (100 g, 25 min, 4 °C, accélération et décélération 2), les algues ont été lavées trois fois par ajout d'eau milli-Q stérile suivi d'une centrifugation (100 g, 10 min, 4 °C, accélération et décélération 2) et retrait du surnageant. Les algues lavées ont alors été reprises dans de l'eau milli-Q stérile et placées en erlenmeyer dans un incubateur INFORS où la température et la luminosité sont maintenues constantes (22 ± 1 °C et 90 ± 10 µmol/m²/s) et l'aération est assurée par agitation (100 rpm).

### Quantification des algues

Trois échantillons de 4 à 5 mL de la suspension d'algues lavées ont été filtrés à travers des filtres pré-tarés en nitrocellulose de taille de pores 1,2 µm (réf. 512-0267, VWR). Les filtres ont été séchés pendant 30 min à 70 °C, puis ont été pesés pour déterminer une concentration en matière sèche apparente. La mesure de cette valeur a permis, par proportionnalité, de prévoir la concentration en matière sèche effective de la suspension et par conséquent de diluer cette dernière de manière à obtenir une concentration de 0,50 ± 0,1 g/L de matière sèche. La concentration en matière sèche de la suspension après dilution a été contrôlée par séchage de trois échantillons de 4 à 5 mL à 100°C pendant 24h, suivi d'une pesée du résidu.

### 7.1.3. Expériences de captage des métaux par les algues

### Exposition des algues aux métaux

Pour chaque expérience, 30 mL de suspension d'algues lavées ont été introduits dans des erlenmeyers de 100 mL. La concentration des algues est de 0,5 ± 0,1 g/L de matière sèche. Le pH a ensuite été ajusté à sa valeur de consigne par ajout de KOH ou de HCl concentré. Les échantillons ont été placés dans l'incubateur INFORS pendant une nuit pour adapter les algues au pH. A t₀, 300 µL de solution de LnCl₃ (Ln = le lanthanide considéré), 100 fois plus concentrée que la concentration finale souhaitée, ont été introduits dans les échantillons. Les quantités introduites ont été contrôlées par pesée. Le pH a été réajusté quotidiennement à sa valeur de consigne.

### Suivis physiologiques

L'état physiologique des algues a été suivi via la mesure de la densité cellulaire et du rendement de photosynthèse.

### Prélèvement de surnageant

Un échantillon de 1,2 mL de suspension d'algues a été prélevé. Le surnageant a été séparé des algues par deux centrifugation successives (100 g, 10 min, 4 °C, accélération et décélération 3) (centrifugation, prélèvement du surnageant puis centrifugation de ce surnageant). Après la dilution de 1 mL de surnageant dans 4 mL de HNO₃ 1,25 % (chaque dilution étant contrôlée par pesée), la solution résultante a été conservée à 4°C dans l'attente d'un dosage ultérieur des métaux en solution par ICP-MS.

### Prélèvement de culot

Un échantillon de 3 mL de suspension d'algues a été prélevé. Les algues ont été récupérées par centrifugation (500 g, 10 min, 4 °C, accélération et décélération 4). Elles ont ensuite été lavées 1 à 2 fois par reprise dans 4 ml d'eau milli-Q, centrifugation (500 g, 10 min, 4 °C, accélération et décélération 4) et retrait du surnageant. Le culot d'algues a été conservé à 4°C dans l'attente d'une minéralisation et du dosage des métaux incorporés par ICP-MS.

### 7.1.4. Dosage des métaux

### Minéralisation des algues

Les algues ont été minéralisées à sec dans 1,5 mL d'eau régale (HNO₃ 65 % / HCl 30 % 2/1 v/v) à 180°C. Le résidu a été repris dans 1 mL de HNO₃ 10 % (solution préparée par dilution d'une solution de HNO₃ 65 % ultra-pur disponible dans le commerce) puis dilué par 10 avec de l'eau milli-Q stérile. Chaque dilution a été contrôlée par pesée.

### Dilution et dosage des métaux

Les solutions issues des prélèvements de surnageant ou de la minéralisation des algues ont été diluées dans HNO₃ 1% de manière à obtenir une concentration en métal comprise dans la gamme étalon. Ces échantillons ont été dosés par ICP-MS par comparaison avec une gamme étalon réalisée à l'aide de solutions fournies par Analab. Chaque dilution a été contrôlée par pesée. La concentration en métal a été calculée comme la moyenne des mesures réalisées sur les différents isotopes.

La solution mère de terre rare utilisée pour l'exposition des algues a également été dosée.

Pour chacun des éléments analysés, l'étendue de la gamme étalon et les isotopes mesurés sont résumés dans le Tableau .

**Tableau VIII : Paramètres d'analyse par ICP-MS**

| Elément | Gamme étalon | Isotopes analysés |
|---|---|---|
| Néodyme | 0,5 à 50 nM | ¹⁴²Nd, ¹⁴⁴Nd et ¹⁴⁶Nd |
| Europium | 0,5 à 10 nM | ¹⁵¹Eu et ¹⁵³Eu |
| Gadolinium | 0,1 à 10nM | ¹⁵⁶Gd, ¹⁵⁸Gd et ¹⁶⁰Gd |
| Terbium | 0,2 à 20 nM | ¹⁵⁹Tb |

Idéalement, le dosage des métaux a été réalisé dans les surnageants dans le cas d'échantillon montrant un pourcentage d'accumulation élevé et dans les culots lorsque de faibles pourcentages d'accumulations ont été observés.

### 7.1.5. Préparation des complexes Ln[citrate]

Les complexes du citrate ont été préparés par mise en solution dans l'eau milli-Q d'une quantité équimolaire en métal et acide citrique. D'après les données thermodynamiques, le complexe se forme alors spontanément.

### 7.2. Fixation des quatre terres rares gadolinium, neodyme, europium et terbium sous forme libre et sous forme complexee par coccomyxa ccap 216/26 dans les conditions optimales pour la bio-epuration (10-6 m de metal, ph 6)

### 7.2.1. Expériences réalisées

L'accumulation de quatre terres rares (Gd, Nd, Eu et Tb) sous deux formes chimiques (cation et complexe du citrate) par *Coccomyxa* CCAP 216/26 a été testée dans les conditions optimales pour le pourcentage d'accumulation : 10⁻⁶ M de terres rares, pH élevé. Les conditions de pH et les concentrations initiales ont été optimisées à l'aide de plans d'expérience composites centrés, appliqués à une gamme de pH comprise entre pH 2 et pH 9 et une gamme de concentration comprise entre 10⁻²M et 10⁻⁶ M de métal. Les optimaux ainsi obtenus, identiques pour deux éléments (Gd et Nd) et deux formes chimiques (cation et complexe du citrate), ont été généralisés.

Les plans d'expériences ont révélé un pH optimal de 6 pour les cations et de 9 pour le complexe du citrate. Cependant, l'influence du pH sur l'accumulation du complexe n'est que très faible et les accumulations à pH 4, 6 ou 8 ne sont pas significativement différentes. Par conséquent, lors de l'expérience présente, tous les tests ont été effectués à pH 6 ± 1, 10⁻⁶ M de terres rares.

Les huit expériences correspondantes ont été réalisées en parallèle et répétées trois fois sur trois semaines et trois biomasses différentes, afin d'estimer la fiabilité intermédiaire.

### 7.2.2. Suivis physiologiques

L'état physiologique des algues a été suivi par mesure du rendement de photosynthèse et de la densité cellulaire (Figure 8). Les algues restent dans un excellent état physiologique : la densité cellulaire augmente et le rendement de photosynthèse reste proche de 60 %, même après 6 jours d'exposition aux terres rares dans les conditions présentes.

### 7.2.3. Suivi de l'accumulation des métaux

Les métaux accumulés ont été dosés dans les surnageants afin de déterminer les pourcentages d'accumulation (%). Les valeurs obtenues sont présentées dans le tableau IX et la Figure 9.
- Les pourcentages d'accumulation sont du même ordre de grandeur pour toutes les terres rares : compris entre 90 et 95 %.
- Pour un même élément, il n'y a pas de différence significative entre l'accumulation du cation ou du complexe du citrate après 24h de contact avec les algues.
- La quasi-totalité des cations de terres rares accumulés par les algues sont captés en moins d'1h dans les conditions d'exposition 10⁻⁶ M de métal, pH 6. Il n'y a plus, ou très peu de variation par la suite, jusqu'à 24h de contact.
- Au moins 3h de contact sont nécessaires pour l'accumulation des terres rares sous forme de complexe du citrate dans les conditions 10⁻⁶ M de métal, pH 6. Le pourcentage d'accumulation continue d'augmenter faiblement entre 3h et 24h de contact.

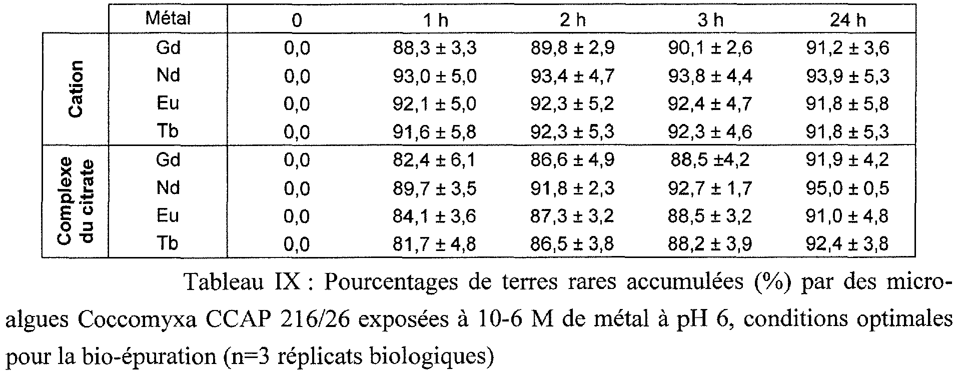

### 7.3. Fixation des quatre terres rares gadolinium, néodyme, europium et terbium sous forme libre et sous forme complexée par Coccomyxa CCAP 216/26 dans les conditions optimales pour le captage des métaux (10-2 M de métal, pH 2)

### 7.3.1. Expériences réalisées

L'accumulation de quatre terres rares (Gd, Nd, Eu et Tb) sous deux formes chimiques (cation et complexe du citrate) par *Coccomyxa* CCAP 216/26 a été testée dans les conditions optimales pour les quantités accumulées : **10⁻² M de terres rares, pH 2.**

### 7.3.2. Suivis physiologiques

L'état physiologique des algues a été suivi par mesure du rendement de photosynthèse et de la densité cellulaire (Figure 10). La croissance des algues exposées à pH 2, en présence ou non de terres rares, est totalement stoppée. Le rendement de photosynthèse décroît légèrement en 6 jours d'exposition à 10⁻² M de terres rares à pH 2. Il reste supérieur à 50 % après 2 jours d'exposition, ce qui témoigne d'une bonne viabilité des algues lors des premières 48h.

### 7.3.3. Suivi de l'accumulation des métaux

Les métaux ont été dosés dans les culots d'algues après minéralisation à l'eau régale, afin de déterminer les quantités de terres rares accumulées (µmol/g de matière sèche). Les valeurs obtenues sont présentées dans le tableau X et la Figure 11.

Dans l'ensemble, les quantités de terres rares accumulées sont relativement proches. Quel que soit l'élément ou la forme chimique considérée, la quantité de métal accumulée est comprise entre 40 et 100 µmol/g de matière sèche après un temps de contact de 3h ou 24h. Dans le détail, on observe de petites différences.
- Dans les conditions d'exposition à 10⁻² M de métal à pH 2, il existe une différence significative d'accumulation entre les différents éléments chimiques. Le gadolinium et le néodyme semblent plus facilement captés que l'europium et le terbium (101 et 78 µmol/g de matière sèche de métal captés en 24h pour le Gd³⁺ et le Nd³⁺ respectivement ; 43 et 39 µmol/g de matière sèche de métal captés en 24h pour Eu³⁺ et Tb³⁺ respectivement).
- Dans le cas du gadolinium et du néodyme, l'accumulation des cations semble un peu plus efficace que celle des complexes du citrate. Les maxima d'accumulation des cations sont atteints en 24h de contact. Quel que soit l'état de la biomasse, la répétabilité est bonne jusqu'à 24h (Figure 11). A 48h de contact, la toxicité peut commencer à se faire sentir selon l'état de la biomasse. L'accumulation des complexes du citrate se fait rapidement durant les 3 premières heures de contact, puis l'augmentation est plus progressive jusqu'à 48h d'exposition.

- Dans le cas de l'europium et du terbium, la différence d'accumulation entre les deux formes chimiques ne semble pas significativement différente. La grande majorité des métaux captés par les algues le sont en seulement 3h. Peu de variations sont visibles par la suite.

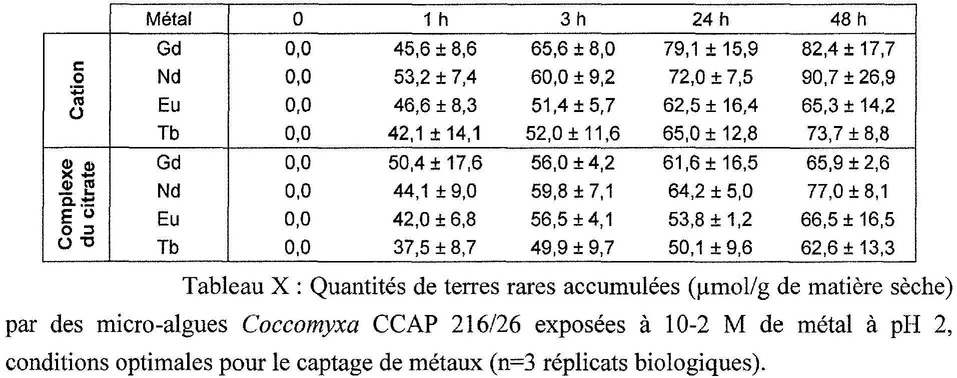

L'utilisation de la forme cationique des terres rares, plus rapidement captée par les algues dans le cas de certains éléments et moins coûteuse en réactifs, semble donc préférable dans les conditions optimales pour la récupération de métaux.

### 7.4. Fixation de l'ion Gd3+ par Coccomyxa CCAP 216/26 dans des conditions de pH plus faibles (pH 1)

### 7.4.1. Expériences réalisées

Afin de tester la fixation des terres rares au plus près des conditions rencontrées dans les solutions de dissolution des déchets urbains, l'accumulation du Gd³⁺ par *Coccomyxa* CCAP 216/26 a été testée dans des conditions de pH plus faible (pH 1) que celles testées jusqu'à présent :
- 10⁻² M de Gd³⁺ et pH 2
- 10⁻² M de Gd³⁺ et pH 1

Les deux expériences ont été réalisées en parallèle.

### 7.4.2. Suivis physiologiques

L'état physiologique des algues a été suivi par mesure du rendement de photosynthèse et de la densité cellulaire (Figure 12). La croissance des algues exposées à pH 1 et 2, en présence et en l'absence de terres rares, est fortement ralentie. Le rendement de photosynthèse est impacté par les conditions d'expositions testées ici, mais les algues sont encore viables à 48h et 6 jours lors des expositions à 10⁻²M de Gd³⁺, pH 1. Après 6 jours d'exposition, le rendement de photosynthèse des algues exposées à 10⁻² M de Gd³⁺ et pH 2 reste proche de 50 %. Le rendement de photosynthèse d'algues exposées à 10⁻² M et pH 1 chute en dessous des 20 % en 6 jours.

### 7.4.3. Suivi de l'accumulation des métaux

Les métaux ont été dosés dans les culots d'algues après minéralisation à l'eau régale, afin de déterminer les quantités de terres rares accumulées (µmol/g de matière sèche). Les valeurs obtenues sont présentées dans le Tableau XI et la Figure 12.

Les algues sont capables d'accumuler du Gd³⁺, même lorsqu'elles sont exposées pendant 48h à un pH très faible (pH 1). Les quantités accumulées sont plus élevées à pH 2 qu'à pH 1.

La plus forte accumulation est observée après 24h d'exposition des algues à 10⁻² M de Gd³⁺ et pH 2. Une quantité proche de 100 µmol/g de matière sèche est alors captée par les algues. Cette condition reste la condition optimale pour le captage des métaux.

**Tableau XI : Quantités de terres rares accumulées (Unité arbitraire) par des micro-algues Coccomyxa CCAP 216/26 exposées à 10-2 M de métal et à pH 1 ou 2 (n=3 réplicats biologiques dans les conditions 10-2 M de Gd3+ et pH 1 ; n=2 réplicats biologiques dans les conditions 10-2 M de Gd3+ et pH 2).**

| Concentration | pH | 0 | 1 h | 3 h | 24 h | 48 h |
|---|---|---|---|---|---|---|
| 10⁻² M | 1 | 0,0 | 34,6 ± 11,6 | 33,8 ± 15,1 | 41,2 ± 14,2 | 65,9 ± 31.0 |
| 10⁻² M | 2 | 0,0 | 35,1 ± 2,1 | 35,4 ± 1,0 | 47.0 ± 0,4 | 58,6 ± 0,2 |

## Revendications

1. Algue verte unicellulaire du genre *Coccomyxa,* **caractérisée en ce qu'**il s'agit de la souche de *Coccomyxa* déposée le 29 janvier 2013 auprès de la CCAP sous le numéro CCAP 216/26.

2. Procédé de captage d'au moins un élément, radioactif ou non, choisi parmi Cs, Ag, Co, Mn, Sr, Cu, Cr, Zn, Ni, Fe, Sb,U, les actinides, les lanthanides et terres rares, telles que Sc, Y, La, Ce, Pr, Nd, Pm, Sm, Eu, Gd, Tb, Dy, Ho, Er, Tm, Yb, Lu, et/ou au moins un des radioisotopes ¹⁴C et ³H, à partir d'un milieu aqueux contenant ledit élément et/ou ledit radioisotope en solution, **caractérisé en ce que** ledit captage est effectué en incubant, dans ledit milieu aqueux, des algues vertes unicellulaires du genre *Coccomyxa* telles que définies dans la revendication 1.

3. Procédé selon la revendication 2, **caractérisé en ce que** ledit élément est choisi parmi Sr et Cu.

4. Procédé selon l'une quelconque des revendications 2 ou 3, **caractérisé en ce que** ledit milieu aqueux est un milieu radioactif.

5. Procédé selon l'une quelconque des revendications 2 ou 3 **caractérisé en ce que** ledit milieu aqueux est un milieu non radioactif.

6. Procédé selon la revendication 4, pour le captage d'un métal choisi parmi Ag, Co, Cs, U, Mn, Cu et Sr, **caractérisé en ce que** ledit métal est sous forme d'isotope radioactif, ou sous forme de mélange d'isotopes.

7. Procédé selon l'une quelconque des revendications 2 à 6, **caractérisé en ce que** lesdites algues vertes sont associées à au moins un autre microorganisme et/ou au moins une plante multicellulaire.

8. Procédé selon l'une quelconque des revendications 2 à 7, **caractérisé en ce que** la croissance desdites algues vertes telles que définies dans la revendication 1 est maîtrisée en contrôlant l'illumination dudit milieu aqueux.

9. Procédé selon une quelconque des revendications 2 à 8, **caractérisé en ce que** l'élément capté, radioactif ou non, est choisi parmi Cs, Ag, Co, Mn, Sr, Cu, Cr, Zn, Ni, Fe, Sb, les actinides, les lanthanides et terres rares, telles que Sc, Y, La, Ce, Pr, Nd, Pm, Sm, Eu, Gd, Tb, Dy, Ho, Er, Tm, Yb, Lu, et **en ce que** ledit procédé comprend une étape de récupération dudit élément à partir des algues.

10. Procédé selon une quelconque des revendications 2 à 9, **caractérisé en ce que** le pH du milieu aqueux est compris entre 1 et 6.

11. Utilisation d'une algue verte du genre *Coccomyxa* telle que définie dans la revendication 1, pour la dépollution d'un milieu aqueux contenant au moins un élément, radioactif ou non, choisi parmi Cs, Ag, Co, Mn, Sr, Cu, Cr, Zn, Ni, Fe, Sb, les actinides, les lanthanides et terres rares, et/ou au moins un des radioisotopes ¹⁴C et ³H.

12. Utilisation selon la revendication 11 **caractérisée en ce que** ledit milieu est radioactif.

13. Utilisation selon l'une des revendications 11 ou 12, **caractérisée en ce que** ledit élément est choisi parmi Sr et Cu.

14. Utilisation selon l'une quelconque des revendications 12 ou 13, **caractérisée en ce que** ladite algue verte est associé à au moins un autre microorganisme radiorésistant ou radiotolérant et/ou au moins une plante multicellulaire radiorésistante ou radiotolérante.

## Patentansprüche

1. Einzellige Grünalge der Gattung *Coccomyxa,* **dadurch gekennzeichnet, dass** es sich um den Stamm von *Coccomyxa* handelt, der am 29. Januar 2013 bei der CCAP unter der Nummer CCAP 216/26 hinterlegt wurde.

2. Verfahren zum Einfangen mindestens eines radioaktiven oder nicht radioaktiven Elements, ausgewählt aus Cs, Ag, Co, Mn, Sr, Cu, Cr, Zn, Ni, Fe, Sb, U, den Aktiniden, den Lanthaniden und seltenen Erden wie Sc, Y, La, Ce, Pr, Nd, Pm, Sm, Eu, Gd, Tb, Dy, Ho, Er, Tm, Yb, Lu, und/oder mindestens eines der Radioisotope ¹⁴C und ³H aus einem wässrigen Milieu, das das Element und/oder das Radioisotop in Lösung enthält, **dadurch gekennzeichnet, dass** das Einfangen durch Inkubation der einzelligen Grünalgen der Gattung *Coccomyxa* nach Anspruch 1 in das wässrige Milieu erfolgt.

3. Verfahren nach Anspruch 2, **dadurch gekennzeichnet, dass** das Element aus Sr und Cu ausgewählt ist.

4. Verfahren nach einem der Ansprüche 2 oder 3, **dadurch gekennzeichnet, dass** das wässrige Milieu ein radioaktives Milieu ist.

5. Verfahren nach einem der Ansprüche 2 oder 3, **dadurch gekennzeichnet, dass** das wässrige Milieu ein nicht radioaktives Milieu ist.

6. Verfahren nach Anspruch 4 für das Einfangen eines Metalls, das aus Ag, Co, Cs, U, Mn, Cu und Sr ausgewählt ist, **dadurch gekennzeichnet, dass** das Metall in Form eines radioaktiven Isotops oder in Form einer Isotopenmischung vorliegt.

7. Verfahren nach einem der Ansprüche 2 bis 6, **dadurch gekennzeichnet, dass** die Grünalgen mindestens einem anderen Mikroorganismus und/oder mindestens einer vielzelligen Pflanze zugeordnet sind.

8. Verfahren nach einem der Ansprüche 2 bis 7, **dadurch gekennzeichnet, dass** das Wachstum der Grünalgen nach Anspruch 1 durch Steuerung der Beleuchtung des wässrigen Milieus beherrscht wird.

9. Verfahren nach einem der Ansprüche 2 bis 8, **dadurch gekennzeichnet, dass** das eingefangene radioaktive oder nicht radioaktive Element aus Cs, Ag, Co, Mn, Sr, Cu, Cr, Zn, Ni, Fe, Sb, den Aktiniden, den Lanthaniden und seltenen Erden wie Sc, Y, La, Ce, Pr, Nd, Pm, Sm, Eu, Gd, Tb, Dy, Ho, Er, Tm, Yb, Lu ausgewählt ist, und dass das Verfahren einen Schritt der Rückgewinnung des Elements mittels der Algen umfasst.

10. Verfahren nach einem der Ansprüche 2 bis 9, **dadurch gekennzeichnet, dass** der pH des wässrigen Milieus zwischen 1 und 6 inklusive ist.

11. Verwendung einer Grünalge der Gattung *Coccomyxa* nach Anspruch 1 für die Reinigung eines wässrigen Milieus, das mindestens ein radioaktives oder nicht radioaktives Element enthält, ausgewählt aus Cs, Ag, Co, Mn, Sr, Cu, Cr, Zn, Ni, Fe, Sb, den Aktiniden, den Lanthaniden und seltenen Erden, und/oder mindestens eines der Radioisotope ¹⁴C und ³H.

12. Verwendung nach Anspruch 11, **dadurch gekennzeichnet, dass** das Milieu radioaktiv ist.

13. Verwendung nach einem der Ansprüche 11 oder 12, **dadurch gekennzeichnet, dass** das Element aus Sr und Cu ausgewählt ist.

14. Verwendung nach einem der Ansprüche 12 oder 13, **dadurch gekennzeichnet, dass** die Grünalge mindestens einem anderen strahlenresistenten oder strahlentoleranten Mikroorganismus und/oder mindestens einer strahlenresistenten oder strahlentoleranten vielzelligen Pflanze zugeordnet ist.

## Claims

1. Unicellular green alga of the genus *Coccomyxa,* **characterized in that** it is the strain of *Coccomyxa* deposited on 29 January 2013 with the CCAP under number CCAP 216/26.

2. Method for capturing at least one element, whether or not radioactive, selected from among Cs, Ag, Co, Mn, Sr, Cu, Cr, Zn, Ni, Fe, Sb, U, actinides, lanthanides and rare earths such as Sc, Y, La, Ce, Pr, Nd, Pm, Sm, Eu, Gd, Tb, Dy, Ho, Er, Tm, Yb, Lu and/or at least one of the radioisotopes ¹⁴C and ³H, from an aqueous medium containing said element and/or said radioisotope in solution, **characterized in that** said capturing is performed by incubating, in said aqueous medium, unicellular green algae of the genus *Coccomyxa* such as defined in claim 1.

3. Method according to claim 2, **characterized in that** said element is selected from among Sr and Cu.

4. Method according to any of claims 2 or 3, **characterized in that** said aqueous medium is a radioactive medium.

5. Method according to any of claims 2 or 3, **characterized in that** said aqueous medium is a non-radioactive medium.

6. Method according to claim 4, to capture a metal selected from among Ag, Co, Cs, U, Mn, Cu and Sr, **characterized in that** said metal is in the form of a radioactive isotope, or in the form of a mixture of isotopes.

7. Method according to any of claims 2 to 6, **characterized in that** said green algae are associated with at least one other microorganism and/or at least one multicellular plant.

8. Method according to any of claims 2 to 7, **characterized in that** the growth of said algae such as defined in claim 1 is controlled by controlling the illumination of said aqueous medium.

9. Method according to any of claims 2 to 8, **characterized in that** the captured element, whether or not radioactive, is selected from among Cs, Ag, Co, Mn, Sr, Cu, Cr, Zn, Ni, Fe, Sb, actinides, lanthanides and rare earths such as Sc, Y, La, Ce, Pr, Nd, Pm, Sm, Eu, Gd, Tb, Dy, Ho, Er, Tm, Yb, Lu, and **in that** said method comprises a step to recover said element from the algae.

10. Method according to any of claims 2 to 9, **characterized in that** the pH of the aqueous medium is between 1 and 6.

11. Use of a green alga of the genus *Coccomyxa* such as defined in claim 1, to depollute an aqueous medium containing at least one element, radioactive or not, selected from among Cs, Ag, Co, Mn, Sr, Cu, Cr, Zn, Ni, Fe, Sb, actinides, lanthanides and rare earths and/or at least one of the radioisotopes ¹⁴C and ³H.

12. Use according to claim 11, **characterized in that** said medium is radioactive.

13. Use according to one of claims 11 or 12, **characterized in that** said element is selected from among Sr and Cu.

14. Use according to any of claims 12 or 13, **characterized in that** said green alga is associated with at least one other radioresistant or radiotolerant microorganism and/or at least one radioresistant or radiotolerant multicellular plant.
